# EUROPEAN PATENT SPECIFICATION

(11) **EP 0 995 802 B1**
(45) Date of publication and mention of the grant of the patent: **23.12.2009**
(21) Application number: 99401687.1
(22) Date of filing: 06.07.1999
(51) Int. Cl.: C12N 15/86, C12N 15/48, C12N 5/10

(54) **Method for the delivery of nucleic acids to cells in vitro or ex vivo**
Verfahren zur Verabreichng von Nukleinsäuren in Zellen in vitro oder ex vivo
Procédé pour l'apport des acides nucléiques dans les cellules in vitro ou ex vivo

(30) Priority: 10.07.1998 US 113280
(43) Date of publication of application: 26.04.2000
(73) Proprietor: UNIVERSITE PIERRE ET MARIE CURIE, 75252 Paris Cédex 05 (FR)
(72) Inventor: Lemoine, Francois, 75011 Paris (FR); Klatzmann, David, 75013 Paris (FR); Movassagh, Mojgan, 75017 Paris (FR); Salzmann, Jean-Loup, 75011 Paris (FR)
(74) Representative: Becker, Philippe

(56) References cited:
- WO-A-00/55343
- WO-A-97/14809
- US-A- 5 017 492
- MOVASSAGH M ET AL: "HIGH-LEVEL GENE TRANSFER TO CORD BLOOD PROGENITORS USING GIBBON APE LEUKEMIA VIRUS PSEUDOTYPE RETROVIRAL VECTORS AND AN IMPROVED CLINICALLY APPLICABLE PROTOCOL" HUMAN GENE THERAPY,XX,XX, vol. 9, no. 2, 20 January 1998 (1998-01-20), pages 225-234, XP000867251 ISSN: 1043-0342
- GALLARDO H F ET AL: "Recombinant retroviruses pseudotyped with the vesicular stomatitis virus G glycoprotein mediate both stable gene transfer and pseudotransduction in human peripheral blood lymphocytes." BLOOD, vol. 90, no. 3, 1997, pages 952-957, XP002152873 ISSN: 0006-4971
- DATABASE MEDLINE [Online] US NATIONAL LIBRARY OF MEDICINE (NLM), BETHESDA, MD, US; BAUER T R JR ET AL: "Transduction of human hematopoietic cells and cell lines using a retroviral vector containing a modified murine CD4 reporter gene." retrieved from STN Database accession no. 97200256 XP002152878 & HUMAN GENE THERAPY, (1997 FEB 10) 8 (3) 243-52. ,
- SEKI T ET AL: "The human Thy-1 gene: structure and chromosomal location" PROCEEDINGS OF THE NATIONAL ACADEMY OF SCIENCES OF USA,US,NATIONAL ACADEMY OF SCIENCE. WASHINGTON, vol. 82, October 1985 (1985-10), pages 6657-6661, XP002119746 ISSN: 0027-8424
- JOHNSON GARY D ET AL: "Expression of meprin subunit precursors: Membrane anchoring through the beta subunit and mechanism of zymogen activation." JOURNAL OF BIOLOGICAL CHEMISTRY, vol. 269, no. 10, 1994, pages 7682-7688, XP002152874 ISSN: 0021-9258
- DATABASE BIOSIS [Online] BIOSCIENCES INFORMATION SERVICE, PHILADELPHIA, PA, US; December 1997 (1997-12) STAQUET MARIE J ET AL: "Fibronectin upregulates in vitro generation of dendritic Langerhans cells from human cord blood CD34+ progenitors." Database accession no. PREV199800048246 XP002152879 & JOURNAL OF INVESTIGATIVE DERMATOLOGY, vol. 109, no. 6, December 1997 (1997-12), pages 738-743, ISSN: 0022-202X
- GHOSH MADHUMITA ET AL: "Truncating alpha-helix E' of p66 human immunodeficiency virus reverse transcriptase modulates RNase H function and impairs DNA strand transfer." JOURNAL OF BIOLOGICAL CHEMISTRY, vol. 270, no. 13, 1995, pages 7068-7076, XP002152875 ISSN: 0021-9258
- JACQUES PAMELA S ET AL: "Modulation of HIV-1 reverse transcriptase function in "selectively deleted" p66/p51 heterodimers." JOURNAL OF BIOLOGICAL CHEMISTRY, vol. 269, no. 2, 1994, pages 1388-1393, XP002152876 ISSN: 0021-9258
- LIANG CHEN ET AL: "The roles of the human immunodeficiency virus type 1 pol protein and the primer binding site in the placement of primer tRNA3Lys onto viral genomic RNA." JOURNAL OF VIROLOGY, vol. 71, no. 12, December 1997 (1997-12), pages 9075-9086, XP002152877 ISSN: 0022-538X
- MOVASSAGH M ET AL: "High level of retrovirus-mediated gene transfer into dendritic cells derived from cord blood and mobilized peripheral blood CD34+ cells." HUMAN GENE THERAPY, (1999 JAN 20) 10 (2) 175-87. , XP000960663
- YANG S ET AL: "Generation of retroviral vector for clinical studies using transient transfection." HUMAN GENE THERAPY, (1999 JAN 1) 10 (1) 123-32. , XP000960660
- DARDALHON V ET AL: "Green fluorescent protein as a selectable marker of fibronectin-facilitated retroviral gene transfer in primary human T lymphocytes." HUMAN GENE THERAPY, (1999 JAN 1) 10 (1) 5-14. , XP000960664

## Description

The present invention relates to methods and constructs for the delivery of nucleic acids to cells in vitro or ex vivo, such as hematopoietic cells. The invention relates more particularly to retroviral-mediated gene delivery to hematopoietic cells in vitro or ex vivo, with high efficiency; and disclosed are vector constructs and cells used in this method.

### BACKGROUND AND INTRODUCTION.

The use of recombinant retroviruses is one of the most successful methods to introduce a nucleic acid into hematopoietic cells. This gene transfer strategy has been proved to be useful for both basic research and clinical applications. Thus, in humans, much progress has been made for the transduction of hematopoietic progenitors (Hughes et al., 1989; Moore et al., 1992; Nolta et al., 1992; Moritz et al., 1993; Lu et al., 1993a; Hatzfeld et al., 1996) or mature lymphocytes (Bunnell et al., 1995). However, it turns out that some efforts are still necessary to improve the transduction of hematopoietic cells (HC), particularly in view of therapeutical applications. To improve the stability, durability and efficiency of gene transfer into a large amount of HC, several parameters can be modified, including 1) the cycling of target cells; 2) high virus-titers 3) a stable integration and expression of the transgene; 4) transduction procedures preserving the initial function of transduced cells without any toxicity on the proliferation and differentiation; 5) development of transduction protocols that are in accordance with good medical procedures.

Although direct contact between hematopoietic cells and viral packaging cell lines has been shown to increase the efficiency of retroviral-mediated gene transfer into these cells comparatively to infection with viral supernatant (Moritz et al., 1993), this method is not suitable for clinical applications. Indeed, one danger in performing direct retroviral-mediated gene transfer using cocultures is the contamination of the transduced cells by virus-producing cells and therefore the risk of in vivo transfer of virus-producing packaging cells in recipients given transduced cells. To eliminate this risk, one alternative strategy has been to use a transwell coculture system in which virus-producing packaging cells are separated from target cells by a porous membrane (Germeraad et al., 1994). Other strategies using viral supernatant have been also developed for the transduction of different cell types such as human T lymphocytes and CD34⁺ cells, aimed to increase either contact between viral particles and target cells (Moore et al., 1992; Moritz et al., 1994; Bunnell et al., 1995; Chuck and Palsson, 1996) or to increase the expression of amphotropic virus receptors (Crooks and Kohn, 1993; Bunnell et al., 1995). Thus, it has been shown that fibronectin (FN) improved the transduction efficiency of murine and human hematopoietic progenitors (Moritz et al., 1994; Hanenberg et al., 1996) due to colocalization of retrovirus and target cells on specific FN fragments (Moritz et al., 1996). Recently, Bunnell et al have also shown that centrifugation at low-temperature incubation increased the transduction of human and nonhuman primate T lymphocytes (Bunnell et al., 1995).

Another approach to increase the transduction efficiency is to perform a selection of the infected cells, using for instance a marker gene co-expressed by the retroviral vector.

The invention now provides a novel in vitro or ex vivo method of nucleic acid delivery to hematopoietic cells, with very high efficiency and low toxicity. The invention can be used to prepare transduced HC in vitro or ex vivo, which are suitable for either basic research applications (i.e., gene regulation studies, screening methods, etc.), recombinant protein production, or in vivo applications in humans or animals (vaccines, grafts, etc.).

More particularly, the instant invention resides in
1. A method of delivery of a nucleic acid to hematopoietic cells in vitro or ex vivo, wherein said method comprises:
   a) providing a population of hematopoietic cells,
   b) contacting said population of hematopoietic cells with a defective recombinant retrovirus, wherein said retrovirus:
      - is pseudotyped with a GALV envelope, and
      - comprises said nucleic acid,
   c) subjecting the cell population to a centrifugation step; and
   d) collecting the cell population obtained in step c),
   wherein the retrovirus further comprises:
   - a marker nucleic acid, coding for a membrane polypeptide comprising an extracellular domain anchored in or at the membrane, but lacking an intracytoplasmic domain;
   - a bicistronic unit comprising said nucleic acid and said marker nucleic acid operably linked by an IRES sequence:
   and wherein said defective recombinant retrovirus is produced in a packaging cell line comprising a truncated retroviral pol DNA;
2. The method of 1, wherein, in c), the cell population is subjected to a centrifugation step and a fibronectin adhesion step;
3. The method of 1 or 2, wherein, between (c) and (d), the cell population is contacted again with a defective recombinant retrovirus as defined in step (b);
4. The method of 1, wherein said membrane polypeptide is human Thy-1;
5. The method of 1 or 4 wherein said membrane polypeptide comprises a tag;
6. The method of 1, wherein the centrifugation is carried out in the presence of a polycation, such as polybrene or protamine sulfate;
7. The method of 1 or 6, wherein the centrifugation is performed between 100 to 3000 g;
8. The method of 2, wherein the fibronectin adhesion step is performed by incubating the cell population on a support coated with human fibronectin or fragments thereof;
9. The method of 1, wherein the hematopoietic cells are immature hematopoietic cells;
10. The method of 1, wherein said hematopoietic cells are selected from T lymphocytes, B lymphocytes, monocytes and dendritic cells;
11. The method of 9, wherein after step (d) said hematopoietic stem cells are further differentiated into dendritic cells.

Thus, the above preferred embodiment offers the possibility of transducing different T-cell subsets using clinical grade reagents and a 24-hour infection at levels that will avoid, for most clinical applications, further selection or expansion of transduced T-cells.

As explained above, the general method according to this invention combines several advantageous features, such as the use of a pseudotyped retrovirus, a centrifugation and a fibronectin adhesion steps. As illustrated in the examples these combinations of features act in synergy to provide a very high level of transduction of hematopoietic cells (near 100%), even without a selection step. Also, the claimed method can be used to transduce any hematopoietic cells of mammalian origin, i.e., human or animal, such as immature or mature hematopoietic cells. In this regard, as shown in the examples, the method according to the present invention is particularly suited for delivering nucleic acids to immature hematopoietic cells, such as hematopoietic progenitor and stem cells. The claimed method is particularly efficient for the delivery of nucleic acids to hematopoietic CD34+ stem cells.

Furthermore, the method of this invention is also particularly suited for gene delivery to mature hematopoietic cells, such as for instance T lymphocytes (including helper T lymphocytes, cytotoxic T lymphocytes, natural killers, lymphokine-activated killers, etc), B lymphocytes, monocytes and dendritic cells or hematopoietic tumor cells (i.e., B lymphoma cells, for instance). As illustrated in the examples, very high transduction levels have been obtained, following the method of this invention, with dendritic cells and mature T lymphocytes, which confirm the wide range of applicability of the instant method.

In the method of this invention, the population of hematopoietic cells can be provided by any technique known by the skilled artisan. These techniques include blood or other tissue sample collection (spleens or nodes) and isolation of the relevant cell population by any routine technique (gradients, centrifugations, chromatography, cell sorting, etc). The cells can be either prepared just before use in the present method, or can be cells available in cell collections and libraries. As indicated above, these cell populations can be of mammalian origin, preferably of human or animal origin. Furthermore, for the purpose of the instant invention, it is preferred that the cell population comprises at least 50% of hematopoietic cells, more preferably above 65%. In a most preferred embodiment, the cell population is highly enriched and comprises at least 80%, preferably at least 90% of hematopoietic cells. These cells are generally cultured in any appropriate medium known to the skilled artisan.

As mentioned above, the instant invention uses as the nucleic acid delivery vehicle a retrovirus. Recombinant retroviruses have been disclosed and used in the art for many applications, including in vitro, ex vivo and in vivo gene delivery. Their structure and production are well known to those skilled in the art. Very briefly, most recombinant retroviruses are created by replacing, in the recombinant genome, the viral genes gag, pol and env with a nucleic acid of interest, and then produced in a so-called packaging cell, which produces the complementing functions encoded by gag, pol and env. Examples of such packaging cell lines are, for instance PA317, PsiCRIP or Am12. The recombinant retrovirus can be created using different types of retroviruses, such as MoMLV (Moloney Murine Leukemia Virus) ALV, BLV, MMTV or RSV for instance, or using lentiviruses such as HIV, SIV or CAEV, for instance. Finally, since the tropism of a retrovirus is determined essentially by the envelope protein (ENV), most of the packaging cells contain a gene encoding an amphotropic envelope protein, i.e., a protein that confer on the virus the capacity to infect most mammalian cells, including human cells. Very often, the envelope is the A4070 amphotropic envelope.

However, the inventors have now found that the use of a different envelope protein could, in combination with the other characteristics of the instant method, improve significantly the efficiency of transduction of hematopoietic cells. More particularly, in the present invention, the retrovirus that is being used is pseudotyped with an envelope of the Gibbon Ape Leukemia Virus (GALV). The term pseudotyped means that the envelope protein and the other gag and pol proteins of the recombinant retrovirus are of different origins. Accordingly, the retrovirus that is being used in the instant method comprises an ENV protein of a GALV virus. As demonstrated in the examples, the presence of such GALV envelope instead of an amphotropic retroviral envelope provides, in combination with the other process features, a very high level of transduction of hematopoietic cells, close to 100%, without any selection step and with no apparent toxicity.

To prepare such pseudotyped retroviruses, it is convenient to use a packaging cell line that contains the GALV env gene, such as the packaging cells TE-FLY GA18 disclosed by Cosset et al. (Cosset et al., 1995). Of course, any other cell expressing the GALV envelope protein and Mo-MLV-based gag and pol genes could be used in a similar way to produce pseudotyped retroviruses, as discussed in the experimental section.

Furthermore, in a particular embodiment of the instant invention, the cell line that is used to produce the defective recombinant retrovirus is a packaging cell line comprising a truncated retroviral pol DNA. More particularly, the packaging cell comprises a truncated retroviral pol DNA which lacks any overlapping sequence with the env coding region. As explained below, the use of such packaging cell potentially increases the safety of the production method. These overlapping sequences could allow some recombination event to take place, which may affect the genetic stability of the packaging cells as well as the quality of the recombinant retroviruses. The inventors have now shown that it is possible to prepare a truncated retroviral pol DNA, that lacks or has reduced overlapping sequence with env, and still remains biologically active.

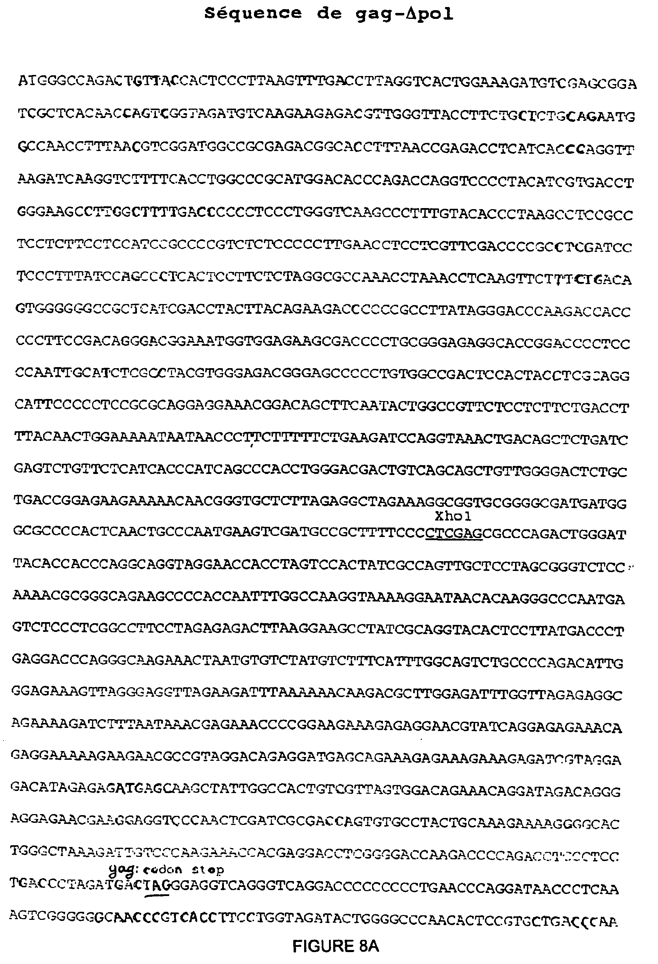

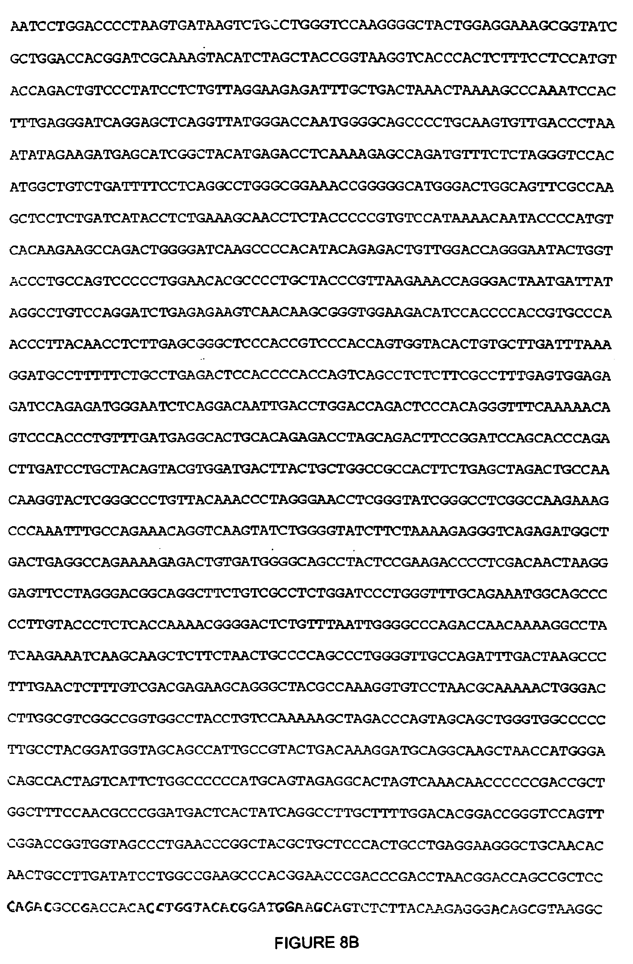

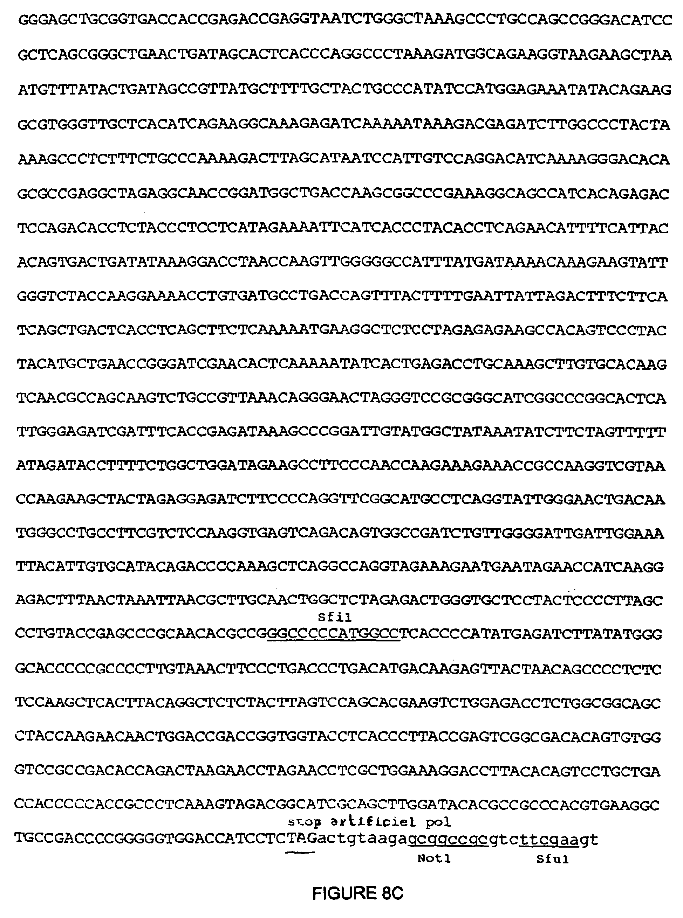

Also disclosed herein therefore is a nucleic acid coding for a biologically active retroviral POL protein lacking between 3 to 50 amino acid residues at the C-terminal end, at least. Preferably, the nucleic acid lacks 80% at least of the overlapping sequences with the env coding region, more preferably at least 90%. An example of the 3' end of such a nucleic acid is GGACCATCCTCTAG (SEQ ID NO: 1). An example of a nucleic acid sequence of the invention encoding a functional truncated POL protein is represented in Figure 8 (in particular in residues from the gag stop codon to the pol artificial stop codon). Any variant of this sequence is also contemplated in particular any variant in which nucleic acid residues have been modified without affecting the encoded protein (due to the degeneracy of the genetic code) as well as other variants with modified residues that still retain the biological activity of POL. Such variants include truncated POL encoding nucleic acids prepared from other retroviral strains, in particular from MoMLV, MMTV or RSV retroviruses. Such variants also include mutants of the above sequence comprising preferably less than 10%, more preferably less than 5%, advantageously less than 3% of modified amino acids.

Also disclosed herein is a genetically modified cell that comprises a DNA encoding a truncated retroviral pol DNA. More preferably, such cell is a retrovirus packaging cell that further comprises a retroviral gag gene and a gene encoding an envelope protein.

The recombinant retrovirus used in the invention further comprises a marker nucleic acid, coding for a membrane polypeptide. Accordingly, the recombinant retrovirus comprises a first nucleic acid whose delivery to the cells is sought (the nucleic acid) and a second nucleic acid (the marker nucleic acid) encoding a membrane polypeptide that allows detection of the transduced cells.

The marker nucleic acid codes for a membrane polypeptide that comprises an extracellular domain capable of anchoring in the cell membrane, and is devoid of intracytoplasmic domain. The use of such membrane polypeptide as a marker is advantageous in that it does not produce any biological signal within the cell, due to the absence of a functional intracytoplasmic domain, even upon binding of a specific ligand, such as an antibody or the natural ligand, to its extracellular domain. More preferably, the anchoring of the extracellular domain is effected either directly by an interaction of said extracellular domain with membrane proteins or lipids, or by an intramembrane domain, that incorporates into the cytoplasmic membrane.

A specific and advantageous example of such a membrane polypeptide is human Thy-1 (Seki et al., 1985; Planelles et al., 1995). This protein belongs to the superfamily of immunoglobulins and is rather small (about 140 amino acids, 25-30 kDa) which is easily compatible with the cloning capacity of retroviruses. Furthermore, Thy-1 is devoid of an intracytoplasmic domain. Indeed, this molecule interacts with the cell membrane by a glycosylpgosphatidylinositol (GPI) binding through its C-terminal end. Thus, Thy-1 can be used as a marker without further structural modification, which reduces the risks of immunogenicity. Finally, Thy-1 is absent from mature T cells (< 1 %) and dendritic cells, for instance, and can be easily detected by monoclonal antibodies. For instance, the anti-Thy-1 monoclonal antibody produced by hybridoma K117 can be used (ATCC accession number HB-8553). This molecule therefore represents a very convenient marker for transduced hematopoietic cells.

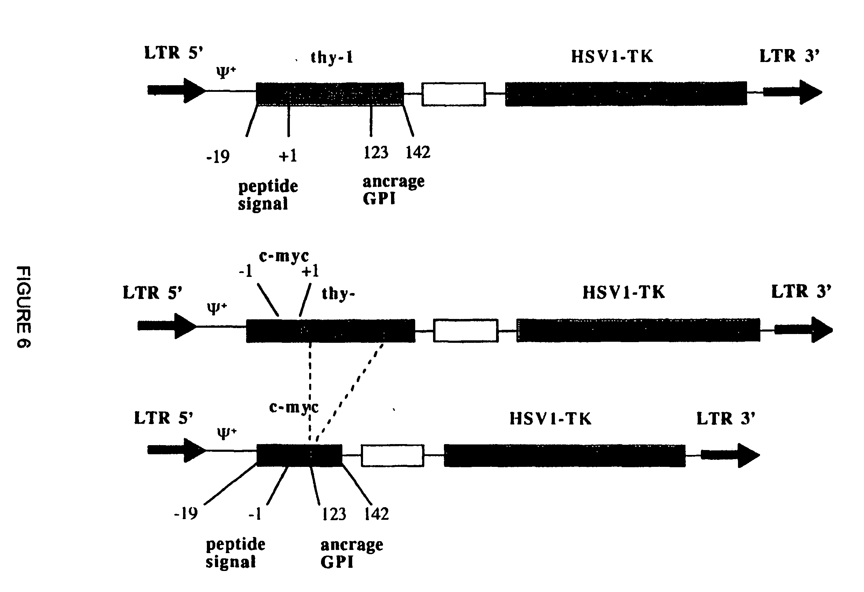

Furthermore, the marker polypeptide can be further modified by introduction of a "tag" sequence, i.e., a short sequence that can be easily detected. An example of such a tag is the c-myc tag having the amino acid sequence EQKLISEEDL, corresponding to residues 410 to 419 of human c-Myc protein. Such a tag can be introduced in or fused to any marker gene, in particular to the above marker gene, either intact or deleted (see Figure 6). In a particular embodiment, the marker gene is a chimeric gene comprising a tag and residues for anchoring in or at the plasma membrane. More particularly, the marker gene comprises a signal peptide, a tag and the residues for GPI or intracytoplasmic anchor. Advantageously, the marker polypeptide of the instant invention can be any polypeptide
- devoid of a functional intracytoplasmic domain,
- capable of anchoring at or into the cell membrane, and
- comprising a "tag" sequence.

Again, an example of such a molecule is a hybrid comprising the human Thy-1 protein and the c-myc tag sequence, at its N-terminal end (tag/Thy-1). Another example is a hybrid molecule comprising a portion of the Thy-1 protein that is responsible for cell membrane anchoring (this portion comprises essentially the C-terminal end of Thy-1, for instance the last 20 to 50 residues at the C-terminal end of Thy-1) linked to a tag sequence.

The nucleic acid of interest and the marker nucleic acid are part of a bicistronic unit that comprise said nucleic acid and said marker nucleic acid operably linked by an IRES sequence. This is the case for the following reasons:
- a bicistronic unit generally requires less cloning space than two separate transcriptional units and thereforen allow the insertion of potentially larger genes.
- the organization as a bicistronic unit ensures that where the transduced cells express the marker protein, they will also express the nucleic acid of interest. This is particularly important because, although most markers allow selection of the cells that effectively contain the nucleic acid of interest, they provide no information as to the expression of said nucleic acid in the cells. The bicistronic unit that is present in the retrovirus of this invention enables, if needed, the selection of transduced cells and ensures that the selected cells indeed express the nucleic acid of interest.

Accordingly, although the transduction efficiency of the method according to this invention is very high and does not require a selection step, the presence of this marker nucleic acid, as a bicistronic unit, can be used to control the quality of the cell population or to trace the cells after in vivo administration, if needed.

The invention uses a recombinant retrovirus which is pseudotyped with a GALV envelope and comprises a bicistronic unit comprising, operably linked by an IRES sequence:
- said nucleic acid of interest, and
- said marker nucleic acid encoding a membrane-anchored human polypeptide (e.g., a Thy-1 polypeptide).

The bicistronic unit can be inserted in the same or opposite orientation than the retroviral LTR sequence. It can be under the control of any appropriate promoter, functional in mammalian cells, including the LTR sequence or any other viral or house-keeping promoter, for instance.

In the present method, the contacting between the population of hematopoietic cells and the recombinant retrovirus is accomplished by incubating the cells in the presence of a suspension of the retroviruses. The suspension can be a supernatant of a packaging cell culture producing the virus, or a dilution or concentrate thereof. The suspension can also be a partially purified supernatant, enriched for the viruses, obtained according to known methods (i.e., gradient centrifugation, chromatography or the like). The incubation is generally performed with a suspension of retroviruses comprising between 10⁴ and 10⁷, more preferably between 10⁴ and 10⁶ viral particles, for 10⁴ cells, approximately. It should be understood that the precise amount of viruses per cell used in the method can be adapted by the skilled artisan without undue experimentation.

In accordance with the present invention, following the contacting step, the cell population is subjected to a centrifugation step alone or associated to a fibronectin adhesion step. Indeed, as illustrated in the examples, the combination of a particular type of retrovirus with this specific treatment of the cells allows one to obtain very high transduction levels of hematopoietic cells, in the range of 100%. Although not mandatory, it is best to perform the centrifugation step first and then the fibronectin adhesion step.

The centrifugation can be performed in any suitable device (tube, plate, bags, etc) and with any appropriate centrifuge, preferably under sterile conditions. The centrifugation can be conveniently performed at between 100 to 3000 X g, preferably 300 to 2500 X g. The temperature and time course of the centrifugation can be adapted by the skilled artisan. Typically, the centrifugation is performed at room temperature, for 1 to 5 hours, approximately.

In a particular embodiment of the invention, the centrifugation is carried out in the presence of a polycation, such as polybrene or protamine sulfate. The inventors have indeed found that the addition of such polycations could further increase the transduction efficiency of the present method. The polycations usually induce changes in the membrane permeability to ions, and therefore create an electrochemical alteration of the membranes which may further increase the transduction efficiency. Polybrene can be used for instance at concentrations equal to or below 4µg/ml, at which no toxic effect on hematopoietic cells has been reported. Alternatively, and preferably, the polycation used is protamine sulfate, which has the advantage of being approved for human use.

This centrifugation step can be performed one or several times, in order to further increase, if necessary, the transduction levels. In particular, the centrifugation can be performed from 1 to 5 times, preferably 1 to 3 times.

The fibronectin adhesion step can be carried out by incubating the cell population on any appropriate support coated with fibronectin or fragments thereof. Fibronectin is a complex extracellular matrix molecule with multiple cell adhesion sites that can bind both hematopoietic cells (such as hematopoietic progenitors) and retroviral particles (Moritz et al., 1996). More preferably, in the present method, the fibronectin adhesion step is performed by incubating the cell population on a support coated with human fibronectin or fragments thereof. The support can be any device suitable for cell culture or introduced into a cell culture, such as a plate, microplate, bag, bead, tube, etc. Preferably, the adhesion step is performed by plating the cell population on plates or bags coated with (human) fibronectin or fragments thereof. As indicated above, the fibronectin is preferably of human origin, and the entire molecule can be used or only fragments thereof. The term "fragments thereof" designates any portion of the (human) fibronectin having the capacity to bind both hematopoietic cells and a retroviral particle. Such fragments have been disclosed in the art (such as fragment CH 296-FN, Hanenberg et al., 1996) and can be prepared by routine methods comprising digestion of the protein and assaying the binding capacity of the resulting fragments. Moreover, the fibronectin or fragments can be of recombinant origin, i.e., produced by expression in a recombinant host cell of the corresponding nucleic acid molecule. Conveniently, the fibronectin adhesion step is performed using a support coated with 1 to 500 pmol/cm2 of fibronectin or fragment thereof. Preferred density is between 10 and 100 pmol/cm2. Remarkable effects have been observed with a support coated with about 50 or 75 pmol/cm2 of fibronectin (or fragment thereof). The adhesion step can be carried out over varying periods of time, preferably between 1 to 10 hours, in particular between 2 and 6 hours, 4 hours being a specific example of an efficient condition.

Following the adhesion step, the transduced cells are collected by any suitable method and suspended in fresh medium or buffer. The cells can be used for any suitable application or stored under appropriate conditions.

In a particularly preferred embodiment of this invention, the cell population is contacted twice with the recombinant retrovirus. In this regard, in the method described above, between (c) and (d), the cell population is contacted again with a defective recombinant retrovirus as defined above. As illustrated in the examples, this double infection provides an enhanced gene delivery to the cells, without toxicity. In a more particular embodiment, steps b) and c) of the present method can be repeated several times, preferably 1 to 4, more preferably 1 to 3 times, in order to further improve the transduction levels, if appropriate.

Also disclosed herein is a population of human hematopoietic cells obtained by the method disclosed above. As explained before, these cell populations are suitable for either basic research applications (i.e., gene regulation studies, screening methods, etc.), recombinant protein production, or in vivo applications in humans or animals (vaccines, grafts, etc.). Of particular interest are cell populations comprising immature hematopoietic cells, such as CD34 positive hematopoietic stem cells or dendritic cells, or human T lymphocytes (e.g., CD4+ and/or CD8+ T lymphocytes). As shown in the examples, the present methods enable the production of these cell populations with a transduction efficiency close to 100%, without toxicity and with no selection step or coculture. Furthermore, the transduced immature hematopoietic cells described above can also be used to produce transduced dendritic cells that retain their antigen-presenting capacities.

Further, the transduced hematopoietic stem cells are produced and then further differentiated into dendritic cells.

Also disclosed herein are means to produce transduced dendritic cells by transduction of immature hematopoietic cells, as described above, followed by differentiation into dendritic cells. As shown in the examples, the differentiation can be carried out by culturing the stem cells in the presence of various lymphokines such as GM-CSF, interferon and/or interleukins.

It should be understood that the present disclosed method may be applicable to other cell populations, such as, for instance, human tumor cells (i.e., non-hematopoietic tumor cells as well as tumor cell lines) which can be administered following transduction in order to generate or modulate the immune response, or embryonic cells (of murine or human origin for instance) which can be used for basic research.

Other advantages of the present invention will be apparent from the following examples which should be regarded as illustrative only and not limiting the scope of the present invention.

### LEGEND TO THE FIGURES

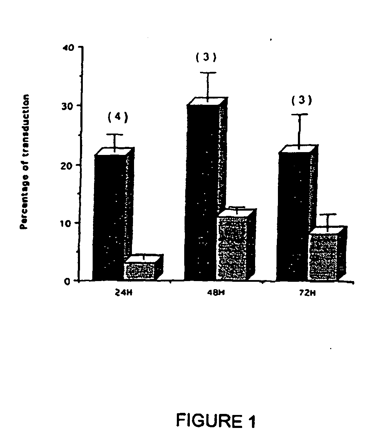

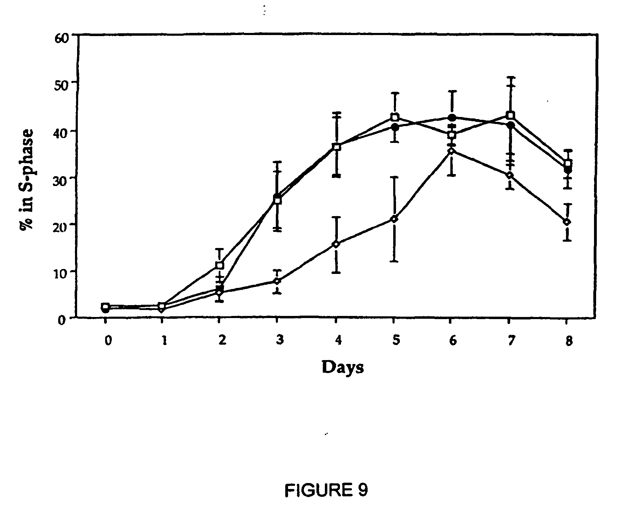

Figure 1 : Comparison of gene transfer efficiency between viral supernatant and direct coculture.
   CD34⁺ cells were infected in the presence of 4µg/ml polybrene either directly by cocultivation with TA7 packaging cells (■) during 1-3 days or with viral supernatant (░) during 1-3 cycles of 24 hours each. After infection, cells were plated for CFC assay. The percentage and total number of transduced CFC were evaluated after X-gal staining of the colonies. Similar infection procedures using the T-Mosalf ecotropic cell line were performed as a negative control. Results are the mean ± SEM of (n) separate experiments.

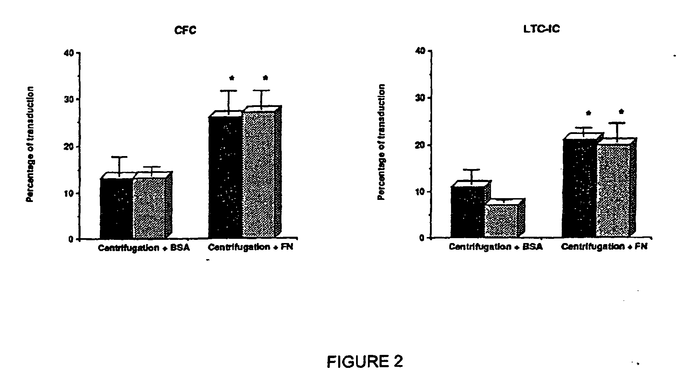

Figure 2 : Effects of fibronectin (Panel A) or centrifugation and fibronectin (Panel B) with (░) or without (■) polybrene on the transduction efficiency of CFC and LTC-IC-derived colonies.
   Panel A: Results are the mean ± sem of 3 separate experiments.
   The number of CFC obtained for 1500 plated CD34⁺ cells was 144±21 and 119±7 in the presence of BSA or BSA + polybrene, respectively. The number of LTC-IC-derived colonies obtained for 5000 CD34+ cells initially plated in LTC was 507±125 and 355±116 in the presence of BSA or BSA + polybrene, respectively.
   Panel B: Results are the mean ± sem of 5 or 3 separate experiments for CFC and LTC-IC, respectively.
   The number of CFC obtained for 1500 plated CD34⁺ cells was 168±40 and 165±40 in the presence of centrifugation + BSA or centrifugation + BSA + polybrene, respectively. The number of LTC-IC-derived colonies obtained for 5000 CD34⁺ cells initially plated in LTC was 693±178 and 510±173 in the presence of centrifugation + BSA + polybrene, respectively. BSA = Boving serum albumin : FN = Fibronectin * p<0.05 (paired t-test).

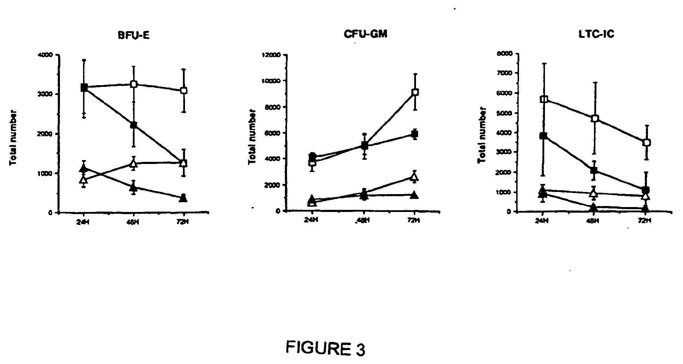

Figure 3 : Total number of CFC and LTC-IC after 24, 48 and 72 hours of infection. CD34+ cells were infected by viral supernatant using centrifugation followed by adhesion to FN in the presence (■, Δ) or absence of Pb (□) for 24, 48 and 72 hours. After infection, cells were plated for CFC and LTC-IC assays. Total number of progenitors (square) and total number of transduced progenitors (triangle) evaluated after X-gal staining of the colonies are shown. Similar infection procedures using the T-Mosalf ecotropic cell line were performed as a negative control. Results are the mean ± SEM of 3 separate experiments. Number of CFC and LTC-derived colonies are calculated for an input of 5.10⁴ CD34⁺ cells.

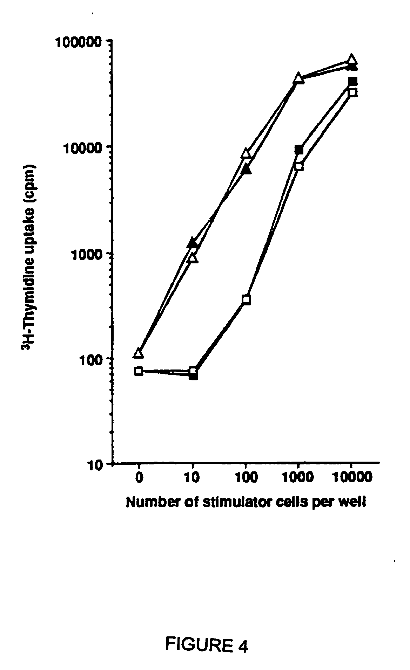

Figure 4 : Allogeneic Mixed lymphocyte reaction (MLR) of CB CD34⁺-derived DC. DC generated from liquid cultures of retrovirally-infected or mock-infected CB CD34⁺ cells were either sorted into transduced or untransduced CD1a⁺ cells (triangle or unsorted (square symbols) before being assayed for MLR. Results from experiments carried out after TE-FLY GA18 viral infection and TE-FLY MOSALF mock-infection are presented. Results are the mean of triplicate wells, PHA-stimulated T lymphocytes (always > 2 x 10⁴ cpm) and T lymphocytes alone (always < 100 cpm) being used as positive and negative controls, respectively.
   A representative MLR performed after sorting of CD1a⁺ cells is shown.
   Black triangle : Transduced CD1 a+ β-gal+ DC;
   White triangle : untransduced CD1a⁺ DC.
   A representative MLR performed with unsorted cells used as a bulk APC population is shown.
   Black square : Transduced cells:
   White square : untransduced cells.
Figure 5 : Representation of Plasmid pKM4 comprising a bicistronic unit.
Figure 6 : Structure of the recombinant retroviral genomes comprising a bicistronic unit.
Figure 7 : Construction of a truncated POL molecule.
Figure 8 : Nucleotide sequence of a GAG-APOL construct.
Figure 9: Kinetics of Cell Cycling.
   Purified CD4⁺ (◊), CD8⁺ (•) and total T-cells (□) were activated in culture using immobilized OKT3 mAb (5 µg/ml) and rh IL-2 (600 Ul/ml) from day 0 to day 8. Results, expressed as the mean ±SEM of at least 3 separate experiments, represent the percentage of each T-cell subset in S-phase.

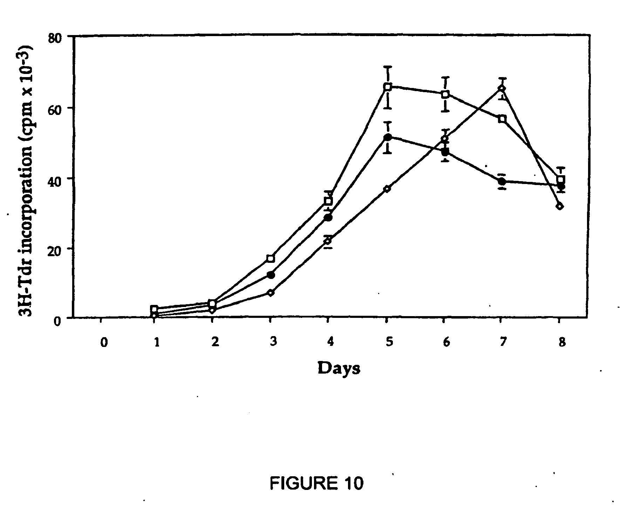

Figure 10: Kinetics of cell proliferation.
   CD4⁺ (◊), CD8⁺ (•) and total T-cells (□) activated as described above in Figure 9 were seeded at 10⁵ cells/well. Cell proliferation was studied by 3H-Tdr incorporation by pulsing T-cells from triplicate wells with ³H-Tdr for an additional 16 hours. Cell proliferation determination was measured using a β-counter. Results represent the mean ±SEM of at least 3 separate experiments.

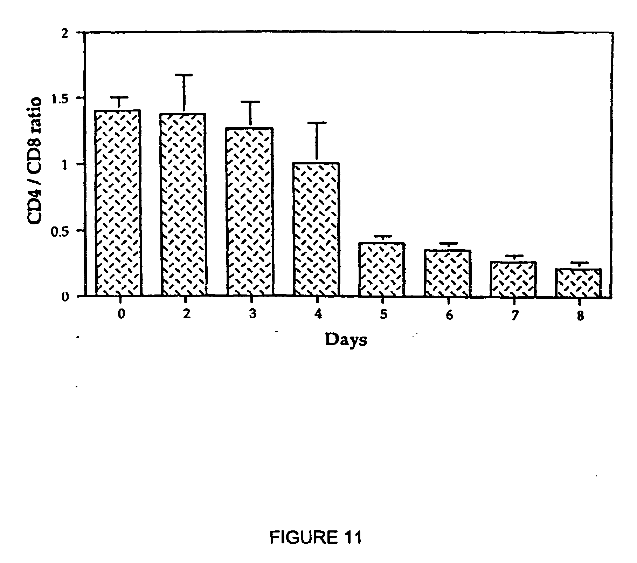

Figure 11: CD4/CD8 ratio of total T-cells ( 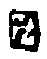 ) cultured from day 0 to day 8.
   Every day, activated T-cells harvested from serial cultures were stained with anti-CD4-PE and anti-CD8-FITC mAbs and analyzed by flow cytometry. Results represent the mean ±SEM of at least 3 separate experiments.

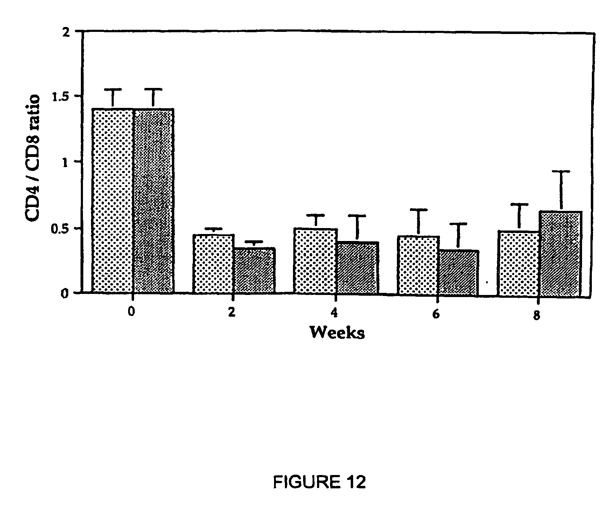

Figure 12: CD4/CD8 ratio in T cell cultures established from total T_{d3}(░) and total T_{d5} ( 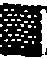 ) transduced cells.
   After retroviral infection, total T-cells were propagated in culture and the CD4/CD8 ratio was determined by flow cytometry every 2 weeks. Results represent the mean ±SEM of 4 separate experiments.

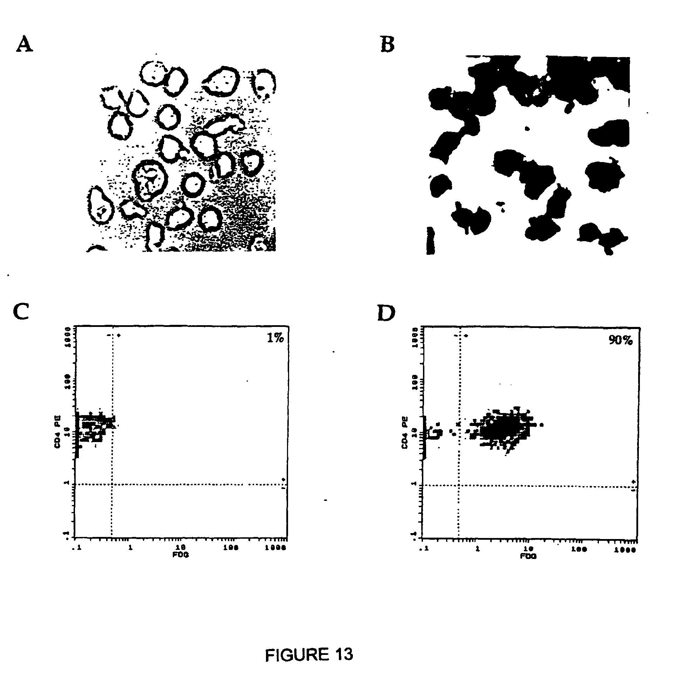

Figure 13: Expression of β-gal activity by transduced T-cells.
   Expression of β-gal activity by transduced T-cells harvested from liquid cultures was determined either by using X-gal staining (Figure 13A and 13B) and/or by FDG staining (Figure 13C and 13D).
   Photomicrograph Fig 13A (magnification X 1,000) and Fig 13C show CD4⁺ cells mock-infected with ecotropic (TE-FLY MOSALF) retroviral cell supernatants. No β-gal activity was detected.
   Photomicrograph Fig. 13B (magnification X 1,000) and Fig 13D show CD4⁺ cells that have been infected with GALV-pseudotyped (TE-FLY GA18) retroviral cell supernatants. Photomicrograph Fig. 13B shows transduced CD4⁺ cells exhibiting specific nuclear β-gal activity revealed by X-gal staining. Similar staining patterns were obtained with CD8⁺ and total T-cells.

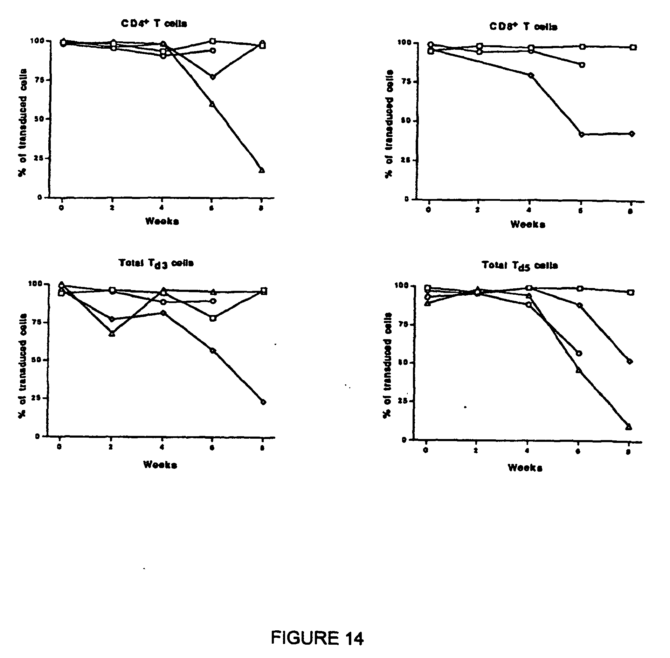

Figure 14: Follow-up of the β-gal gene expression by transduced CD4, CD8, total T_{d3} and total T_{d5} cells propagated in cultures.
   Every two weeks, an aliquot of cultured cells was assayed for β-gal gene expression using X-gal staining and/or FDG staining. Results presented are expressed as percentage transduced cells revealed by X-gal staining. Data from at least 3 separate experiments are shown.

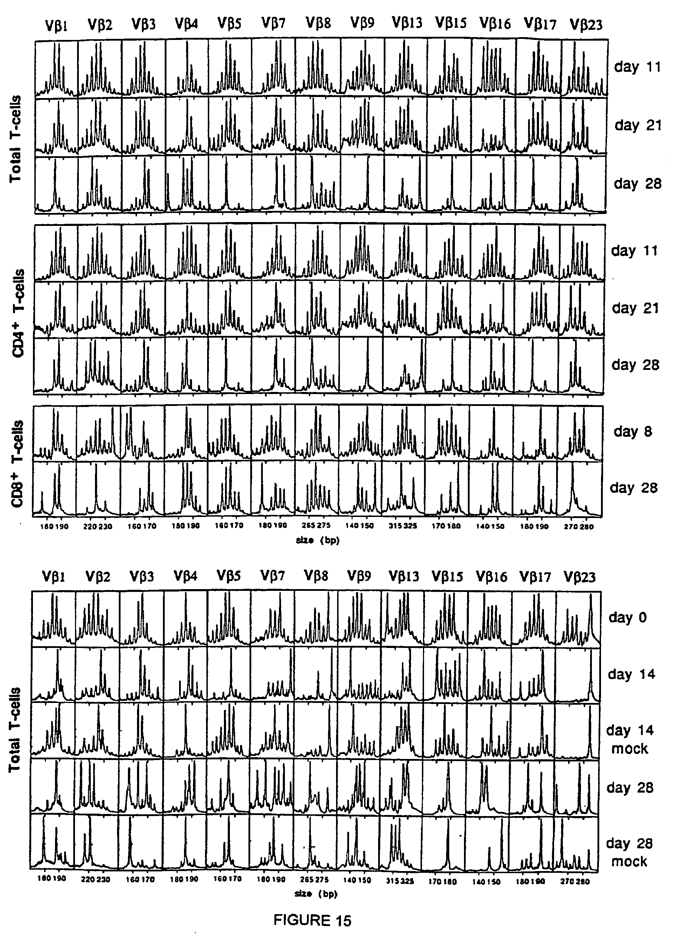

Figure 15: Studies of the Vβ repertoire analysis in mock-infected and transduced T-cells.
   RNA from total T-cells or CD4⁺ or CD8⁺ subsets were reversed transcribed and PCR amplified using a representative set of 13 Vβ primers and a Cβ primer.
   The patterns obtained represent the size and intensity distribution of inframe TCRβ transcripts (horizontal axis, size of amplified products in bp; vertical axis, fluorescence intensity in arbitrary units).
   Data from Experiment 1 (upper panel) and Experiment 2 (lower panel) are representative of 4 independent T-cell cultures from 4 different donors. Day 0 is the day of initiation of the culture. Data from Experiment 1 and from day 14 and day 28 (Experiment 2) are after infection with GALV-pseudotyped retroviral vectors. Mock refers to T-cell repertoire analysis after mock-infection using ecotropic retroviral vectors incapable of infecting human T-cells.

### EXAMPLES

### A. Example 1 . Gene delivery to immature hematopoietic cells (CD34+ cells).

### A1. Materials and methods

### Collection of Cord Blood cells and purification of CD34⁺ cells:

Cord blood (CB) cells, collected according to institutional guidelines, were obtained during normal full-term deliveries. Mononuclear cells (MNC) were separated on Ficoll-Hypaque gradient (density 1.077g/ml), resuspended in Iscove's Modified Dulbecco's Medium (IMDM) and counted. Isolation of CD34⁺ cells was performed as described elsewhere (Movassagh et al., 1996). Briefly, MNC were stained with fluorescein (FITC)-conjugated CD34 monoclonal antibody purchased from Becton Dickinson (Sunnyvale, CA, USA) using a one-step direct immunofluorescent procedure. Unstained cells and cells labeled with conjugated isotype-matched non-specific mouse immunoglobulin conjugated to FITC (Coultronics, Margency, France) were used as negative controls. Then, cells were analyzed and sorted on an EPICS Elite flow-cytometer (Coultronics, Margency, France) on the basis of their light scatter properties and fluorescence intensity. Thus, CD34⁺ cells present within a lymphoid-blast gate, which normally contains the majority of hematopoietic progenitors, were sorted at 1500-2000 cells per second and collected in IMDM containing 50 % fetal calf serum (FCS). In these conditions, the purity of sorted cells, assessed by analyzing an aliquot of cells immediately after sorting, attained more than 96% (range 96-98.5%).

### Retroviral vectors and packaging cell lines:

TA7, T-MOSALF and G18 cells are Mo-MuLV-based packaging cell lines conferring amphotropic, ecotropic, and GALV host ranges, respectively. These cells, kindly supplied by F.L.Cosset (Cosset et al., 1995), derived from a human rhabdomyosarcoma cell line produce higher-titer viruses containing the Escherichia Coli β-galactosidase gene fused with a nuclear localization sequence (nls-LacZ). G18 cells are similar to TA7 cells, except that the amphotropic envelope has been replaced by the GALV envelope. The viral titer determined by infection of NIH-3T3 cells ranged from 5x10⁶ to 10⁷ particles/ml. Cells are cultured in Iscove's modified Dulbecco medium (IMDM) supplemented with L-Glutamine, penicillin/streptomycin and 10% heat-inactivated FCS in a 5% CO₂/95% air humidified incubator.

Viral supernatant, harvested from subconfluent cell monolayers 24 hours after medium change, was filtered through a 0.45 mm low binding protein filter (Costar) prior to transduction experiments.

### Protocols of transduction of CD34⁺ cells:

Purified CD34⁺ cells (3-5x10⁴ cells/ml) were pre-stimulated in IMDM containing 10%FCS, IL-3 (100U/ml, Genzyme, Boston, USA), IL-6 (100U/ml, a gift from Dr L.Aarden, Amsterdam, The Netherlands), SCF (50ng/ml, Amgen, Thousands Oaks, CA, USA), IGF-1 (50ng/ml, Boehringer, Meylan ,France) and bFGF (50ng/ml, Sigma, St Louis, MO, USA) at 37°C for 24 hours as described elsewhere (Movassagh et al., 1997). Then, CD34⁺ cells were infected under similar culture conditions using different protocols denoted as standard protocol, centrifugation protocol, adhesion protocol, optimized protocol: 1) the standard protocol was performed in the presence of 4 mg/ml Pb by infection of stimulated CD34⁺ cells either directly cocultured onto nonirradiated vector-producer cells or with viral supernatant during 24-hours. Cells were infected in 24-well flat bottomed tissue-culture plates (Costar) and maintained at 37°C in a 5% CO₂ incubator; 2) the centrifugation protocol involved exposure of cells to supernatant with or without Pb and centrifugation in round bottomed tubes at 200 X g or 1000 X g at 25°C for 3 hours. Pelleted cells were gently resuspended in 24-well flat bottomed tissue-culture plates, maintained for 4 hours at 37°C in a 5% CO₂ incubator and then reinfected with 1 ml of fresh viral supernatant/well. Thus, 24-hour infection represents two cycles of infection. 3) the adhesion protocol was performed by incubating CD34⁺ cells on plates coated either with optimal concentration (75 pmol/cm²) of human recombinant FN (Moritz et al., 1994) or bovine serum albumin (BSA) at the same concentration as control for 4 hours. Then, cells were infected with or without Pb for 20 hours, viral supernatant being replaced after the 3 first hours; 4) the optimized protocol was performed by combining the last two protocols of infection i.e., centrifugation protocol at 1000 X g for 3 hours, 4-hour-adhesion on FN-coated wells followed by a second infection in the presence or absence of Pb. Infection protocols using supernatant obtained from TA7 cells, and from T-MOSALF cells (mock-infection) were carried out at equal volume of vector supernatant and at similar range of cell concentrations (3-5x10⁴ CD34⁺/ ml). They were performed for 1-3 days, the different procedures being repeated every 24-hours. Thereafter, infected CD34⁺ were counted and plated either in clonogenic methylcellulose progenitor assays and/or in long-term culture (LTC) for LTC-IC assays.

### Clonogenic methylcellulose assays:

For CFC assays, 1500 infected and mock-infected CD34⁺ cells were plated in duplicate methylcellulose cultures as described elsewhere (Movassagh et al., 1996) in 35 mm dishes (Greiner, Frickenhausen FRG), in 1.1 ml of IMDM containing 0.8 % methylcellulose (Fluka 4 000, Buchs, Switzerland), 30 % FCS, 1 % BSA, 100 µM 2-mercaptoethanol (Sigma, Saint-Louis, MO, USA) 2 mM glutamine, 100 U/ml penicillin, 100 mg/ml streptomycin, 3 U/ml recombinant human erythropoietin (rh Epo) (Boehringer, Mannhein FRG), 100 U/ml rh IL-3, 200 U/ml rh granulocyte-macrophage-colony stimulating factor (GM-CSF) (Genetics Institute, Cambridge, MA, USA). Cultures were incubated in a humidified atmosphere containing 5 % CO₂ in air at 37°C. Burst Forming Unit Erythroid (BFU-E) and Colony Forming Unit Granulocyte-Macrophage (CFU-GM) were counted under an inverted microscope, after 16 to 18 days culture.

### LTC-IC assays:

For LTC-IC assays, infected and mock-infected CD34⁺ cells were seeded in 24-well tissue culture plates (Costar) containing LTC medium (StemCell Technologies, Vancouver, BC, Canada) and a pre-established feeder layer of MS-5 cells, a murine stromal cell line supporting normal human hematopoiesis (Isaad et al., 1993). For the establishment of the stromal adherent layer, MS-5 cells were irradiated at 40 Gy and seeded at 4x10⁴ cells/well onto plastic wells precoated with 1% gelatin in order to avoid early detachment of the stromal cell layer. 5,000 CD34⁺ cells/well in 1 ml were cocultured without growth factors or hydrocortisone on MS-5 cells for 5 weeks with weekly half-medium changes. Then, non-adherent and adherent cells obtained after trypsinization in each well were pooled and assayed for their CFC content as described above. The colonies obtained in these secondary methylcellulose cultures are the product of LTC-IC.

### Analysis of transduction efficiency:

Detection of the presence of provirus and nuclear beta-galactosidase activity encoded by the nls-LacZ gene were performed as follows:
a) In methylcellulose assays:
   - X-Gal staining: after counting, colonies were fixed in 1ml PBS containing 1 % glutaraldehyde, 0.2% formaldehyde and 0.02% Nonidet P-40 for 30 minutes, washed twice in phosphate buffer containing 2mM MgCl₂, 0.01% sodium deoxycholate, 0.02% NP40 for 30 minutes and then stained for 24 hours at 37°C with 1 ml of X-gal staining solution containing 5mM ferricyanine, 5mM de ferrocyanine and 1mg/ml X-Gal. This procedure allowed the maintenance of the general morphology of the colonies. The transduction efficiency of the different protocols was evaluated by scoring colonies containing at least 5% of cells expressing β-galactosidase activity under an inverted microscope. Nonspecific staining was easily ruled out by the fact that it was located within the cytoplasm and not in the nucleus.
   - Polymerase chain reaction (PCR): individual colonies (BFU-E, CFU-GM and CFU-M (M=macrophage)) were plucked from the methylcellulose culture medium, and digested with proteinase K in lysis buffer at 56°C for 1 hour followed by 10 minutes exposure at 94°C. The presence of the provirus was determined using the synthetic oligodeoxynucleotide primers complementary to the DNA sequences located within the nls-lacZ gene (5'-CGACTCCTGGAGCCCGTCAGTATC-3') and in the vector upstream of the 3' LTR (5'-GACCACTGATATCCTGTCTTTAAC-3') as described (Ferry et al. 1991). The amplification cycle was 1 min at 94°C, 1 min at 60°C, 1min 30 at 72°C. After 35 cycles, PCR products were run on a 2% agarose gel. The specific 400-bp fragment was detected after ethidium bromide staining under an U.V. light.
b) Flow cytometry: In some experiments, transduction efficiency was directly analyzed by flow-cytometry on CD34⁺ cells 18-24 hours after infection.
Thus, cells were first stained with a phycoerythrin-cyanine 5 (PE-Cy5)-conjugated CD34 monoclonal antibody (Coultronics) using a one-step direct immunofluorescent procedure and then detection of the nuclear beta-galactosidase activity was performed using the fluorescein di-beta galactopyranosidase (FDG) substrate. Briefly, cells were counted and brought to 10⁷ per ml in RPMI containing 2% (vol/vol) fetal calf serum, 10mM Hepes (pH 7.3). 100 ml of 2 mM FDG in H₂O, prewarmed to 37°C were added to 100 ml of solution of cells. Cells were mixed gently but thoroughly and rapidly placed back into a 37°C water bath for 2 min. Then, cells were placed on ice and 1800 ml of ice-chiled isotonic incubation medium were added. The percentage of transduced cells was measured with an EPICS Elite flow cytometer (Coutronics).

### Statistical analysis:

Results are presented as the mean ±SEM of (n) experiments. Comparison of gene transfer efficiency between direct coculture and viral supernatant (standard protocol) was analyzed by using a non-paired t-test. Comparisons of gene transfer efficiencies between the different infection protocols using viral supernatant were analyzed by using a paired t-test. Statistical significance was taken at the 5% level (p<0.05).

### A2. Results.

### Comparison between direct coculture and viral supernatant for gene transfer into CFC:

In a first series of experiments, we have compared the gene transfer efficiency using either direct coculture with amphotropic packaging cell line ( TA7 ) during 1-3 days or infection with viral supernatant during 1-3 cycles of 24 hours each. In both cases, CD34⁺ cells were infected in the presence of Pb after a 24-hour stimulation as described in the material and methods section. The results evaluated on CFC (BFU-E + CFU-GM) are presented in Figure 1. It turned out that when CD34⁺ cells were infected by supernatant, the percentage of transduced CFC was 3±1% after 24 hours and increased significatively (p<0.05) to 11±1% and 8±3% after 48 and 72 hours, respectively. By contrast, when cells were directly infected by cocultivation, higher level of transduction was already obtained after 24-hours. Under the latter condition, 22±3% of the colonies were transduced and the total number of CFC and transduced CFC for 5x10⁴ infected CD34⁺ cells were 4450±1660 and 880±220, respectively. The morphology of transduced colonies was not different from those of non-infected colonies indicating that protein encoded by the nls-LacZ coding sequence was not cytotoxic. No colonies were transduced with T-MOSALF cells that release ecotropic viruses. These data led us to optimize different transduction protocols using viral supernatant.

### Effect of centrifugation, fibronectin and polybrene on the transduction efficiency of CFC and LTC-IC:

We have combined the centrifugation protocol and the adhesion protocol and then evaluated their effects on the transduction efficiency of CFC and LTC-IC. Figure 2 shows that centrifugation + FN, comparatively to centrifugation + BSA, increased by 2-fold (p<0.05) the percentage of transduced CFC and LTC-IC. Under these infection conditions, 26±5% (n=5) and 21±2% (n=6) of CFC and LTC-IC were transduced, respectively. Interestingly, addition of Pb during the adhesion protocol did not further enhance the level of transduction. No significant change of the total number of CFC and LTC-IC was observed.

### Effect of time and number of cycles of infection on the transduction efficiency of CFC and LTC-IC :

We next investigated whether the efficiency of gene transfer might be improved according to time and number of cycles of infection. Thus, CD34⁺ cells were infected by combining centrifugation and adhesion to FN (optimized protocol) with or without Pb for 24, 48 and 72 hours. In the absence of Pb, the results presented in Table 1 and Figure 3 show that the percentage and total number of transduced clonogenic progenitors were significantly increased (p<0.05) using a 48-hour transduction protocol. No difference was observed using a 72-hour transduction protocol, except that the total number of transduced CFU-GM was increased due to an in vitro expansion of these progenitors (see Figure 3). Under these infection conditions, the percentage and total number of transduced LTC-IC remained unchanged. In the presence of Pb, the total number of transduced LTC-IC and BFU-E (Figure 3) was dramatically diminished (p<0.05) after 48 and 72-hours of infection while their percentage (Table 1) only slightly, albeit not significantly, decreased. Because of the time-dependent inhibitory effect of Pb (see figure 3), these data strongly suggest that Pb exerts a toxic effect on both LTC-IC and BFU-E and to a lesser extend on CFU-GM. In order to confirm the toxicity of Pb, CD34⁺ cells were transduced using the standard protocol (i.e. supernatant only) in the presence or absence of Pb during 48 hours. The results of 4 separate experiments show a 32±7% significant inhibition of the total number of CFC in the presence of Pb. In three additional experiments, CD34⁺ cells were infected in the presence of 8 mg/ml protamine sulfate added during all the transduction period (centrifugation + adhesion to FN). It turned out that protamine sulfate, when added to FN, did not further improve the transduction efficiency of hematopoietic progenitors (data not shown). By contrast to Pb, this polycation had no toxic effect. Thus, our results indicate that up to 34±4% of clonogenic progenitors and up to 22±2% of LTC-IC (n=3) can be transduced using a 48-hour infection protocol combining centrifugation at 1000g for 3 hours and adhesion to FN.

### Effect of GALV pseudotyped retroviral vectors on the transduction efficiency of CD34⁺ cells, CFC and LTC-IC:

The Env glycoproteins are responsible for fusion of virus and host cell receptor on the target cell and for fusion of virus and host cell membrane to mediate internalization of the virus. Thus, our objective was to establish whether there were significant differences in the transduction efficiencies using retroviral vectors pseudotyped with GALV envelope in comparison to an analogous amphotropic vector. Therefore, using the 48-hour-optimized protocol described above, CD34⁺ cells were infected with supernatant derived from the G18 cell line. Transduction efficiency was evaluated not only on colonies derived from short and long-term cultures as above but also directly on CD34⁺ cells. FACS analysis on CD34⁺ cells after double staining with FDG and CD34-PE MoAb shows 53±8 % of transduction (Table 2). Transduction efficiency on hematopoietic progenitors shows that b-galactosidase activity was detected in 83±7% of clonogenic progenitors and in 63±4% of LTC-IC-derived colonies (Table 2). The total number of CFC and LTC-IC infected with G18 supernatant remained unchanged comparatively to mock-infected progenitors (not shown). Furthermore, PCR analysis of individual colonies (Table 3) shows integration of the nls-LacZ gene in 98/102 of clonogenic progenitors and 39/40 LTC-IC derived colonies (Table 3). These data demonstrate that very high level transduction of hematopoietic progenitors can be achieved.

### A3. Discussion

CB represents an easily available source of HSC and has the capacity to reconstitute the lympho-hematopoietic system of children as well as of adult recipients after myeloablative therapy (Gluckman et al., 1989). Furthermore, CB cells exhibit in response to various combinations of hematopoietic growth factors high proliferative capacities leading within few weeks to large expansion of cells (Mayani and Lansdorp, 1995). For these reasons, CB has considerable interest as a source of HSC for clinical use (Moritz et al., 1993). The aim of our study was to improve retroviral transduction of CB progenitors under conditions that are suitable for clinical applications meaning a transduction system that do not use direct cocultivation or stromal supportive-cells. Although the latter methods have been proved to efficiently transduce hematopoietic progenitors (Moritz et al., 1993; Nolta et al., 1995), they are not easily applicable for clinical trials because of the risk of infusion of vectors producing cells into patients and because obtaining sufficient quantities of autologous stromal cells can be problematic.

We have developed, using supernatant containing amphotropic retroviral particles, a 48-hour infection protocol combining centrifugation followed by adhesion to FN that allows to reach transduction levels of CB progenitors that are at least as good as using direct cocultivation with vector-producer cells and to maintain the number of progenitors. Using this optimized protocol we have studied, in a second set of experiments, the effects of a pseudotyped retroviral vector carrying the GALV envelope protein on the transduction efficiency. The results showed that very high transduction levels were obtained on both CFC and LTC-IC derived colonies. Thus, our data confirmed, as already reported for T lymphocytes, the advantage of GALV pseudotyped vectors over amphotropic vectors for the transduction of human hematopoietic cells (Bunnell et al., 1995, Porter et al., 1996). Interestingly, we have also studied proviral integration by PCR in individual colonies and showed that almost 100% of them were transduced.

Such differences between proviral integration and transgene expression, are probably due to the fact that β-galactosidase activity detected with X-gal staining is not a very sensitive method. More recently, using the same optimized conditions, we have been able to obtain similar results using mobilized peripheral blood CD34⁺ cells (data not shown). Interestingly, the very high transduction levels we have been able to obtain will also have the advantage to avoid the selection of transduced cells in view of their clinical use.

In summary, we have first optimized , using an amphotropic retroviral vector, a transduction protocol that eliminates the use of cocultivation, extended in vitro exposure to growth factors and/or stromal cells and selection. Then using this optimized protocol and a GALV pseudotyped retroviral vector we have been capable of transducing at very high levels both CFC and LTC-IC under conditions that can be used for clinical applications.

### B. Example 2: Gene delivery to dendritic cells.

Dendritic cells (DC), which are the most potent antigen-presenting cells (APC) (Guéry et al., 1995), can prime resting and naive T lymphocytes and generate memory T-cell responses in vitro and in vivo without additional exogenous adjuvant (Young et al., 1996). With the development of culture conditions for the generation of substantial numbers of DC from either bone marrow, peripheral blood or cord blood (CB) CD34⁺ cells (Romani et al., 1994) as well as from monocytes (Chapuis et al., 1997), these cells have become the ideal vehicle for immunization against infectious agents, malignant cells and even autoreactive lymphocytes. Thus, immunization protocols with either synthetic peptides, intact antigens such as whole virus or tumor cell extracts, unfractionated acid eluted tumor peptides or extracted tumor mRNA to pulse DC exogenously have been proposed (Mayordomo et al., 1995). Using such protocols, eradication of established tumors have been obtained in certain murine models (Zitvogel et al., 1996). Tumor regression has also been observed after vaccination with DC pulsed with lymphoma-specific idiotypes in humans. However, these approaches might be limited by the availability of known immunogenic peptide sequences, by the risk of eliciting T-cell responses against normal tissue antigens or by the dissociation of the peptides from major histocompatibility complex (MHC) molecules.

Therefore, another strategy based on the genetic modification of DC using recombinant defective retroviral vectors can be proposed. Likewise, transduced DC will constitutively express a cDNA encoding an immunogenic antigen that, after endogeneous processing, will generate multiple peptide fragments, some of which are capable of being associated with MHC class I molecules could serve as cytotoxic T cell (CTL) epitopes. By contrast to pulsing, transduction of DC would have several advantages such as a longer term antigen presentation, an induction of immunity in a MHC class I context leading to a specific CTL immune response without knowing immunodominant epitopes and the presentation of tumor or viral-associated antigens in a context of additional immunostimulatory signals not usually provided by most tumor or viral-infected cells. Introducing genes into DC can be realized either directly into mature DC or into CD34⁺ cells that will further differentiate into transduced DC. Although transduction of non-dividing monocyte-derived DC has been recently described by one group (Aicher et al., 1997), retroviral-mediated gene transfer into long-lived CD34⁺ cells that retain a proliferating and differentiating potential into DC would be of a great interest. Thus, different studies have been reported on gene transfer into CD34⁺ dendritic cell progenitors using either cell-free viral supernatants (Bello-Fernandez et al., 1997) or direct cocultivation with producer cell lines (Szabolcs et al., 1997), the latter being not suitable for clinical use. However, the transduction efficiencies obtained to date for human CD34⁺-derived DC remain relatively low in view of inducing relevant clinical T-cell responses.

In order to develop immunotherapy protocols based on genetically modified DC, we have thus investigated the conditions for highly transducing a large amount of DC derived from CD34⁺ cells. CD34⁺ cells were infected either by amphotropic or by gibbon ape leukemia virus (GALV)-pseudotyped retroviral vectors, both of them carrying an easily detectable reporter gene. Then, the effects of various cytokines on the proliferation and differentiation of transduced CD34⁺ into DC as well as their transgene expression were studied. The immunophenotypic and functional properties of transduced CD34⁺-derived DC were compared to non-transduced DC. In addition, by using a semi-solid methylcellulose assay that reveals dendritic progenitors, we have also studied the transduction efficiency into clonogenic dendritic progenitors.

The CRIP packaging cell line producing recombinant retroviruses, with amphotropic host range, carrying the murine CD2 (mCD2) cDNA has been kindly provided by J.M. Heard (CNRS URA 1157, Institut Pasteur, France). Thus, cells transduced with MFG-mCD2 retroviral vectors expressed the mCD2 cell surface antigen that is easily detectable by flow cytometry (Champseix et al., 1996). The NIH-3T3 murine fibroblast cell line was used as negative control.

### B1. Materials and methods

### Collection of CB cells and purification of CD34 cells

CB cells were collected and CD34 cells isolated as described in Example A1.

### Transduction of CD34⁺ cells

Prestimulation and infection of CD34⁺ cells were performed as described in Example A1 with some additional modifications such as the use of Flt-3 ligand (FL) (300ng/ml, Immunex, Seatle, WA, USA) that we have shown in preliminary experiments to improve the transduction level of hematopoietic progenitors from the myeloid lineage (data not shown). Then, CD34⁺ cells (10⁵ cells/ml) were infected for 48-hours under similar culture conditions with viral supernatant harvested from either G18 or MFG-mCD2 cells using the optimized protocol of example A that combined cell centrifugation in the presence of 8 µg/ml protamine sulfate followed by adhesion on wells precoated with 50 µg/cm2 fibronectin fragments (CH 296-FN, Takara Shuzo Ldt, Shiga, Japan). As controls, CD34⁺ cells were mock-infected with either T-MOSALF or NIH-3T3 cell supernatants under the same conditions. Eighteen to twenty four hours after infection, cells were counted, stained in order to determine the percentage of transduction and then selected or not by cell sorting before being cultured for further studies.

### Growth and differentiation of DC from CD34⁺ cells in liquid cultures

Following retroviral infection, cells used either as a bulk population or after selection by cell sorting of FDG⁺ (fluorescein di-beta galactopyranosidase) or mCD2⁺ transduced CD34⁺ cells, were seeded at 2 x 10⁴ cells/ml into 24-well plates (Costar) at 37°C under various culture conditions in order to generate DC. Briefly, transduced and non transduced CD34⁺ cells were cultured in RPMI containing 10% FCS, 1% glutamine, 1% antibiotics, 20 ng/ml granulocyte-macrophage colony-stimulating factor (GM-CSF, Schering Plough, Levallois-Perret, France), 50ng/ml SCF, 50 ng/ml IL-4 (Genzyme) with or without 50U/ml tumor necrosis factor (TNF-a, Genzyme, specific activity 1.6 x 10⁸U/mg) and with or without 50 or 300 ng/ml FL. Culture media were changed at day 5 and day 8 of culture. After 12 day-culture, cells were counted, morphologically characterized, assessed for their transgenic expression, immunophenotyped and tested for their ability to stimulate allogeneic T lymphocytes.

### Clonogenic dendritic progenitors assays (CFU-DC)

For dendritic progenitor assays, 4000 infected or mock-infected CD34⁺ cells were plated in duplicate methylcellulose cultures in 35 mm dishes (Greiner, Frickenhausen FRG) in 1.1 ml of IMDM containing 0.8 % methylcellulose (Fluka 4 000, Buchs, Switzerland), 30 % FCS, 1 % BSA, 100 µM 2-mercaptoethanol (Sigma, Saint-Louis, MO, USA) 2 mM glutamine, 100 U/ml penicillin, 100 µg/ml streptomycin, 50U/ml TNF-a, 50ng/ml IL-4, 20ng/ml GM-CSF, 50 ng/ml SCF and 50ng/ml FL. Cultures were incubated in a 95% humidified atmosphere containing 5 % CO2 in air at 37°C. Colonies (=50 cells) were scored under an inverted microscope after 10-12 days of culture. Colonies containing cells with a typical dendritic morphology (i.e. very elongated and spiny cytoplasmic processes) were counted as CFU-DC. Myeloid colonies were subdivided into granulocyte (CFU-G), macrophage (CFU-M) and granulocyte-macrophage (CFU-GM) subclasses. Colonies containing a mixture of cells with a dendritic and a myeloid morphology were counted as CFU-GM/DC. Transgenic expression and proviral integration were studied on individual colonies by X-Gal staining and by PCR analysis, respectively.

### Transduction efficiency analysis

### a) In liquid cultures:

Retroviral transduction efficiency was assessed both directly on CD34⁺ cells 24 hours after the transduction protocol and on CD1a⁺ cells after 12 days of liquid culture. For cells infected by MFG-mCD2 supernatant, CD34⁺ cells or CD1a⁺ cells were double stained with a FITC-conjugated rat antimurine CD2 mAb (Pharmingen, San Diego, CA, USA) and a PE-Cy5-conjugated CD34 mAb or a PE-conjugated CD1a mAb (both from Coultronics), respectively. As negative controls, infected and mock-infected cells (3T3 cell supernatant) were stained with appropriate irrelevant isotype-matched mAbs. For cells infected by G18 cell supernatant, CD34⁺ or CD1a⁺ cells were first stained with a PE-Cy5-conjugated CD34 mAb or a PE-conjugated CD1a mAb using a one-step direct immunofluorescent procedure and then detection of the nuclear β-gal activity was performed using FDG substrate as described in example A. As negative controls, infected and mock-infected cells (T-MOSALF cell supernatant) were stained with appropriate irrelevant isotype-matched mAbs and FDG substrate. The percentage of transduced cells was determined by flow-cytometric analysis. Then, transduced (mCD2⁺ or FDG⁺ cells) and non-transduced cells (mCD2⁻ or FDG⁻ cells) were sorted for further studies.

### b) In methylcellulose cultures:

Detection of nuclear β-gal activity encoded by the nls-LacZ gene within clonogenic dendritic progenitors using X-Gal staining was performed as described in Example A1.

### c) On cytospin preparations:

Cells were cytospun at 300 X g onto glass slides for 5 minutes, fixed in 1ml PBS containing 1% glutaraldehyde, 0.2% formaldehyde and 0.02% NP40 for 5 minutes, washed twice in PBS and detection of β-gal activity was performed as described above. Then, cells were rinced and saturated with Tris-buffered saline (TBS) containing 0.5% BSA, 0.5% AB serum, 0.02% NP40. After addition of 0.3% H₂O₂ for 2 minutes, cells were rinced again in TBS and incubated in a dark humidified chamber with a CD1a mAb (DAKO, Glostrup, Denmark) for 2 hours at 20°C. A second incubation with peroxidase-labeled antimouse antibodies (DAKO) was then performed for 45 minutes. After washing, peroxidase activity was revealed using diaminobenzidine as substrate (kit Sigma FAST, Sigma). Cells were fixed in absolute ethanol, mounted in glycerol and examined under a light microscope.

### Detection of provirus integration by PCR

PCR and semiquantitative PCR were performed as described in Example A1.

Semiquantitative PCR was performed on sorted and non-sorted CD1a⁺ transduced cells as follows: serial dilutions from cell DNA and from purified plasmid DNA containing the nls-LacZ insert (pMFG nls-LacZ) were amplified by PCR. Then, PCR products were run in a 2% agarose gel and stained with ethidium bromide. The number of copies per cell was determined by comparing the different band intensities.

### Monoclonal antibodies and immunophenotypic analysis of DC

The following murine antihuman mAbs directly conjugated either to fluorescein isothiocyanate (FITC) or to phycoerythrin (PE) were used: CD1a-FITC (Ortho Diagnostic, Raritan, NJ, USA); CD1a-PE, CD14-FITC and CD83-PE (Coultronics); CD54-PE, CD80-PE and HLA-DR-PE (Becton Dickinson, Mountain View, CA, USA); CD40-FITC and CD86-PE (Pharmingen). Negative controls were irrelevant isotype-matched mAbs. After 12 days of culture, cells were double stained with both FITC-conjugated and PE-conjugated mAbs. Briefly, 10⁵ cells were incubated with the appropriate mAbs at 4°C for 20 min, washed two times in PBS/2% of FCS and then fixed in PBS containing 1% paraformaldehyde before analysis by cytofluorography.

### Allogeneic Mixed lymphocyte reaction (MLR)

MLR was performed to test the stimulatory function of transduced DC for allogeneic quiescent T cells comparatively to non-transduced DC after 12 days of culture. MLR were carried out either after selection by cell sorting of FDG⁺ or mCD2⁺ transduced CD1a⁺ cells or using the bulk infected population. Briefly, allogeneic T cells, obtained from normal donor peripheral blood, were isolated by E-rosetting of PBMC with 2-aminoethylisothiouroniumbromid (AET, Sigma)-treated sheep red blood cells and recovered from the pellet after lysis with NH4⁺Cl⁻ buffer (pH 7,2). Irradiated (30 Gys) DC were added in graded doses (E:T ratio varying from 1:10 to 1:10000) to triplicate wells, each containing 10⁵ allogeneic T cells in round-bottomed 96-well tissue culture plates. Cells were maintained in 200µl of RPMI containing 10% human serum at 37°C for 5 days and then pulsed with 1 µCi [³H]-thymidine ([³H]-TdR) for 16 h. [³H]-TdR incorporation into DNA was measured using a beta counter. PHA-stimulated T lymphocytes or T lymphocytes alone were used as positive and negative controls, respectively. Results are expressed as mean counts per minute (mean cpm) ± standard deviation.

### Statistical analysis

Results are expressed as the mean ± SEM of (n) experiments. Statistical analyses were performed using a paired Student t-test unless specified as unpaired t-test. Statistical significance was taken at the 5% level (p< 0.05).

### B2. Results

### Transduction efficiency of CD34⁺ cells

CD34⁺ cells were retrovirally infected with either amphotropic MFG-mCD2 vector or with nls-LacZ GALV-pseudotyped vector using a very efficient 48-hour-transduction protocol (see material and methods). Eighteen to 24 hours after infection, the percentage of total cells and/or CD34⁺ cells expressing the transgenes was determined by flow cytometry after staining of the cells either with mCD2-FITC and CD34-PE-Cy5 mAbs or with FDG substrate and CD34-PE-Cy5 mAbs, respectively. After infection, 87 ± 4% of the cells were still CD34⁺. Results presented in Table 4 showed that the mean percentage of total cells and CD34⁺ cells expressing mCD2 (n=4) were 39 ± 5% and 40 ± 4%, respectively. Higher transduction levels were obtained using GALV-pseudotyped vector, the mean percentage of total cells and CD34⁺ cells expressing β-gal activity (n=4) being 62.5 ± 2% and 72 ± 2 % respectively. Detection of β-gal activity on cell suspensions using X-gal staining gave rise to comparable results than cytofluorographic analysis (data not shown).

### Optimizing the conditions for the generation in liquid culture of DC derived from CD34⁺ transduced cells

We and others have shown that DC can be generated from CD34⁺ cells using GM-CSF, TNF-a, IL-4, SCF and more recently transforming growth factor (TGF)-β and FL (Caux et al., 1992). In order to obtain the highest number of transduced DC, we have studied the effects of FL and TNF-a in combination with GM-CSF, IL-4 and SCF on the dendritic differentiation of genetically engineered CD34⁺ cells. Therefore, transduced mCD2⁺ CD34⁺ cells were sorted after infection and cultured in the presence of GM-CSF + IL4 + SCF ± FL (at 50 or 300 ng/ml) and ± TNF-a for 12 days. Then, cells were studied by double-color flow cytometric analysis, CD1a and mCD2 being used as reference molecules for evaluating the percentage of DC and the percentage of transduction efficiency, respectively. In a first series of experiments, the effects of FL on the dendritic differentiation of mCD2⁺ cells were studied. Results in Table 5 indicate that FL significantly increased the absolute number of CD1a⁺ cells, this effect being due to an increase of the cellularity and not to an increase of the percentage of DC obtained after cultures. Interestingly, the maximum effect of FL was observed at 50 ng/ml. In a second series of experiments, the effects of TNF-a were studied by culturing mCD2⁺ CD34⁺ cells in the presence of GM-CSF, IL4, SCF, FL at 50ng/ml with or without TNF-a. Results (Table 5) showed that TNF-a significantly increased the percentage of CD1a⁺ cells (p<0.05) as well as the coexpression on CD1a⁺ cells of CD80, CD86 and CD54 molecules. By contrast, addition of TNF-a significantly decreased the total number of cells (p<0.05), but the absolute number of CD1a⁺ cells remained unchanged. Similar results were obtained using sorted mCD2⁻ CD34⁺ cells or CD34⁺ cells mock-infected with 3T3 supernatant (data not shown). According to our data, it turned out that the best growth factor combination for obtaining the highest number of transduced DC after 12 days of liquid culture was GM-CSF + IL4 + SCF + TNF-a + FL at 50ng/ml. Under these conditions, 2 x 10⁴ CD34⁺ cells gave rise to an average number of 6.4 ± 2 x 10⁵ CD1a⁺ DC.

### Transgenic expression into DC derived from transduced CD34⁺ cells after 12 days of liquid cultures

In parallel to the data presented above, we have studied the expression of the cell surface marker mCD2 on transduced CD1a⁺ cells. While CD34⁺ cells were sorted according to their mCD2 expression prior to cultures, a decrease of the transgene expression both on total mCD2⁺ cells and on CD1a⁺ mCD2⁺ cells was observed after 12 days of liquid culture. The mean percentage (n=5) of total mCD2⁺ cells and CD1a⁺ mCD2⁺ cells derived from sorted CD34⁺ mCD2⁺ cells were 49 ± 7% and 59 ± 5%, respectively (Table 6A). This diminution was always found whatever the combinations of cytokines used (not shown).

In another set of experiments in which CD34⁺ cells were infected with G18 supernatants, transduced CD34⁺ cells selected by cell sorting on the basis of their β-gal activity (β-gal⁺ cells) were then cultured under our optimized conditions for 12 days. The mean percentage (n=5) of total β-gal⁺ cells and CD1a⁺ β-gal⁺ cells generated from sorted CD34⁺ β-gal⁺ cells were 70 ± 5% and 74.5 ± 4%, respectively (Table 6B). Thus, it appeared that a decrease of the nls-LacZ gene, albeit smaller than for mCD2 gene, was also observed both on total β-gal⁺ cells and on CD1a⁺ β-gal⁺ cells.

In further experiments (n=4), CD34⁺ cells, infected with G18 supernatants, were directly cultured without selection (Table 6B, see total infected CD34⁺ cells). Surprisingly, when gene transfer efficiency was evaluated on DC generated from non-sorted total infected CD34⁺ cells, the percentage of transduced CD1a⁺ cells remained in the same range (73.5 ± 5% of CD1a⁺ β-gal⁺ cells), but the percentage of total β-gal⁺ cells (52.5 ± 3%) was significantly lower (p<0.05).

Overall, it turned out that the percentage of transduced CD1a⁺ cells was significantly higher comparatively to total cells (p<0.05). Furthermore, transduction efficiency of DC was significantly higher when CD34⁺ cells were infected with GALV-pseudotyped vectors (non-paired *t*-test, p<0.05).

In order to study nls-LacZ gene proviral integration, a semiquantitative PCR was performed on cell suspensions obtained after 12 days of liquid cultures from sorted CD34⁺ β-gal⁺ cells (n=4) and from bulk infected CD34⁺ cells (n=4). Comparison of the limiting dilutions of PCR cell products to limiting dilutions of PCR plasmid (positive control) products indicate that cells, whatever their origin (sorted or non-sorted transduced CD34⁺ cells), contained approximatively between 2 and 10 copies of nls-LacZ gene provirus per cell.

These data show that more than 70% of CD1a⁺ cells derived from retrovirally infected CD34⁺ cells can be transduced and have integrated at least 2 proviral copies per cell, 2 x 10⁴ CD34⁺ cells giving rise to an average number of 5 x 10⁵ transduced CD1a⁺ DC cells.

### Transduced CD34⁺-derived DC display normal immunophenotype and have normal capacity to stimulate the proliferation of allogenic T lymphocytes

In order to determine whether retroviral infection and/or expression of mCD2 marker or β-gal activity may affect immunophenotypic differentiation of DC, cells generated either from CD34⁺ mCD2⁺ cells or from CD34⁺ β-gal⁺ cells were analyzed after 12 days of liquid cultures by cytofluorography after double staining with directly conjugated mAbs. Table 7 shows that CD1a⁺ cells transduced either with MFG-mCD2 amphotropic vector or with nls-LacZ GALV-pseudotyped vector were strongly positive for MHC class II (HLA-DR), CD40, CD54, CD80 but expressed low percentage of CD86. CD14 and CD83 molecules were not detected on CD1a⁺ cultured cells (data not shown). Thus, transduced CD1a⁺ DC display immunophenotypic characteristics that are comparable to non-transduced CD1a⁺ cells.

To investigate whether retroviral transduction may alter the functional properties of genetically modified DC, allogeneic MLR assays were performed with transduced (mCD2⁺ or β-gal⁺), non transduced or mock-infected CD1a⁺ cells. Because cell populations are heterogeneous after 12 days of liquid cultures, cell sorting was applied to purify transduced CD1a⁺ mCD2⁺ or β-gal⁺ DC and nontransduced or mock-infected CD1a⁺ cells prior to being assayed in graded doses as stimulatory APC for resting allogeneic T-lymphocytes. The results of a representative experiment out of 3 (Figure 4) demonstrate that transduced CD1a⁺ β-gal⁺ DC had comparable allogeneic T-cell stimulatory capacity than DC derived from mock-infected CD34⁺ cells. Similar results were obtained when transduced CD1a⁺ mCD2⁺ DC were compared to DC derived from non-transduced CD34⁺ cells (n=4, data not shown). Due to the high percentage of DC that can be transduced using nls-LacZ GALV-pseudotyped vectors, the allogeneic T-cell stimulatory capacity of non-sorted transduced cells used as a bulk population was compared to non-transduced cells. Results from a representative experiment out of 3 (Figure 4) indicate that no difference between the bulk transduced CD34⁺-derived DC and the bulk non-transduced CD34⁺-derived DC was observed.

We conclude that DC derived from CD34⁺ cells genetically modified by either a gene coding for a murine cell surface marker or by a gene coding for β-gal activity with nuclear localization remained immunophenotypically and functionally similar to DC derived from non-transduced or mock-infected CD34⁺ cells.

### Transgenic expression into DC colonies derived from transduced CD34⁺ cells in methylcellulose culture assays

We have developed in our laboratory a clonogenic methylcellulose assay allowing the detection of DC colonies which are the cellular product of DC CD34⁺ progenitors (Guigon et al., 1997). These methylcellulose cultures contain more than 65% of dendritic colonies. Therefore, we have studied whether DC CD34⁺ progenitors can be transduced and whether the transgene expression can be maintained during their dendritic differentiation in methylcellulose cultures. For this purpose, bulk CD34⁺ cells retrovirally infected either with G18 supernatants or mock-infected with T-MOSALF supernatants were plated in methylcellulose as described in the material and methods section. After 10-12 days of cultures, colonies were examined under an inverted microscope. Pure dendritic colonies (CFU-DC), mixed myeloid/dendritic colonies (CFU-GM/DC) and myeloid colonies (CFU-GM) were observed. The results presented in Table 8 first indicate that the number of clonogenic progenitors infected with G18 supernatants as well as their morphology (not shown) remained unchanged comparatively to mock-infected progenitors. Second, transduction efficiency into CFU-DC and CFU-GM/DC derived from CD34⁺ cells shows that β-gal activity was detected in more than 90% of them. Third, PCR analysis of individual colonies (Table 8) shows integration of the nls-LacZ gene into more than 95% of dendritic progenitors. Altogether, these data provide evidence, for the first time, that very high level transduction of DC CD34⁺ progenitors can be achieved.

### B3. Discussion

We have studied the transduction of DC derived from CD34⁺ cells infected by two different recombinant defective retroviruses and the growth culture conditions for obtaining a large amount of transduced DC. The two retroviral MFG-derived vectors were a classical amphotropic vector containing a gene coding for the cell surface marker mCD2 and a GALV-pseudotyped vector containing the nls-LacZ gene coding for the β-gal enzyme activity with nuclear localization. Although we did not compare head to head the transduction efficiency of these two vectors, the results of experiments, performed under the same conditions, showed efficiency differences that are dramatic enough to conclude that GALV-pseudotyped vectors gave rise to better transduction levels into CD34⁺ cells than amphotropic vectors.

In the first part of the present study, we have evaluated the effects of FL and TNF-a in combination with GM-CSF, SCF and IL-4 on the generation of DC derived from CD34⁺ cells infected with MFG-mCD2 amphotropic vectors. FL is a synergistic factor, known to enhance the proliferation of very primitive progenitors as well as the myeloid differentiation (Rusten et al., 1996). By adding FL to a growth factor combination already used in our laboratory, we have observed an increase of the total number of both DC and myeloid cells. FL, whose effect was optimal at 50ng/ml, did not increase the percentage of DC nor alter their transduction levels. When cultures were carried out in the presence of TNF-a, the total number of CD1a⁺ cells remained unchanged while the percentage of CD1a⁺ cells increased due to the disappearance of other cell types. Furthermore, costimulatory molecules such as CD80 and CD86 as well as CD54 were upregulated. Overall, it turned out that TNF-a induced the differentiation and maturation of DC, while FL increased their total number by -3 fold as already reported. Thus, the growth factor combination including GM-CSF, IL-4, SCF, FL at 50 ng/ml and TNF-a allows to generate from 2 x 10⁴ CD34⁺ cells an average number of 6.4 x 10⁵ CD1a⁺ cells within 12 days.

One difficulty in view of retroviral-mediated gene therapy applications is the maintenance of an efficient expression level of the transgene during the differentiation of transduced hematopoietic progenitors. Persistent expression is dependent on *i)* the retroviral constructions; *ii)* the functional properties of the promoters into both transduced hematopoietic stem cells and their differentiated cell products; *iii)* the high number of integrated proviral copies into progenitors leading to better chance of maintaining transgene expression throughout the cell differentiation. Therefore, we have studied transgene expression during the dendritic differentiation of sorted transduced CD34⁺ cells. Our results showed a decrease of the transgene expression, that was more pronounced by using amphotropic MFG-mCD2 vector than nls-LacZ GALV-pseudotyped vector in DC generated after 12 days of liquid cultures. However, the level of expression was still relatively high as the mean percentage of transduced DC using MFG-mCD2 or nls-LacZ GALV were 59 ± 5% and 74.5 ± 4%, respectively. Interestingly, the transduction level within DC generated from unsorted total CD34⁺ cells, after infection with nls-LacZ GALV-pseudotyped vector, remained also high after 12 days of liquid cultures, 73.5 ± 5% of DC being β-gal-positive. Under these conditions, a -25 fold expansion of transduced DC could be obtained from total infected CB CD34⁺ cells. By comparison to the results published by other groups that have used either cell-free viral supernatants or direct cocultivation with producer cell lines, we have been able to transduced DC at levels that have never been reached before. Such transduction levels can be explained both by almost 100% infection of CD34⁺ cells and by the relatively large number of nls-LacZ gene copies per cell detected by PCR and that can be expressed under our culture conditions. Thus, using a clinically applicable protocol (i.e., used of both viral supernatants and clinical grade reagents) for the transduction of CD34⁺ cells, we have been able to obtain transduction levels (> 70%) of CD1a⁺ DC that would be sufficient enough to avoid further selection of transduced DC in view of inducing relevant clinical T-cell responses.

Second, we have demonstrated that DC genetically modified to express either a cell surface antigen (i.e. mCD2) or an enzymatic nuclear activity (i.e. β-gal), that can serve as models for viral- or tumor-associated antigen, remained immunophenotypically and functionally identical to untransduced DC. These findings, that are in agreement with data obtained either with murine or human retrovirally transduced DC (Szabolcs et al., 1997), are of importance in view of the clinical use of DC for genetic immunotherapy.

Furthermore, we have developed a semi-solid methylcellulose assay for dendritic progenitors. In addition to the fact that this assay allows quantitation of the number of dendritic progenitors present within a CD34⁺ cell population, we have shown, for the first time, that a very high transduction level (= 94 % of transduction efficiency) can be achieved within these progenitors.

In summary, we have shown that using a GALV-pseudotyped retroviral vector and a combination of growth factors including GM-CSF + TNF-a + IL-4 + SCF and FL, very high level transduction of both CB CD34⁺-derived DC and clonogenic dendritic progenitors can be achieved. Under these conditions, transduced DC expressed normal immunophenotype and potent T-cell stimulatory capacity. Altogether these data are of interest in view of developing immunotherapy protocols based on genetically modified DC.

### C. Example 3 : Gene delivery to T lymphocytes.

As a further illustration of the efficacy of the invention, the delivery method as described above was used to transfer nucleic acids into mature T lymphocytes. Mature CD4+ T lymphocytes were isolated according to known techniques and cultured 3 to 6 days in RPMI medium containing 10% bovine serum albumin, in the presence of OKT3 (5 µg/ml) and IL-2 (600 U/ml). Transduction was performed according to the centrifugation protocol of Example A with supernatant of GALV-pseudotyped retroviruses and 5x105 cells/ml. The cells were centrifuged three times in 24 hours (2 hours at 2400g) at 8, 13 and 20 hours post culture (representing 3 infection cycles). Cells were suspended in fresh medium and transduction efficiency was determined as described in examples A and B.

The results are presented in Table 9, and clearly demonstrate very high levels of transduction, essentially above 90%.

These results further confirm the efficiency of the claimed method for the delivery, in vitro or ex vivo, of nucleic acids to hematopoietic or other cells.

### D. Example 4 : Construction of a retroviral vector comprising bicistronic unit composed of a transgene and a membrane marker gene.

This example discloses the construction of a retroviral vector comprising a bicistronic unit comprising, operably linked by an IRES (Internal Ribosome Entry Site) sequence:
- a transgene: a nucleic acid encoding the HSV-1 TK enzyme.
- a membrane marker: a nucleic acid encoding a human thy-1 protein, either intact, or comprising a tag sequence (the c-myc tag) and/or comprising a deletion in the extracellular domain.

In this example, the bicistronic unit is placed under the control of a retroviral promoter (the 5' LTR sequence), although any other promoter can be used.

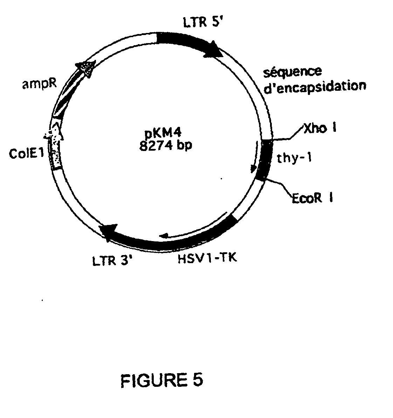

### D1: Construction of Plasmid pKM4 (Figure 5)

Plasmid pKM4 was constructed by conventional recombinant DNA techniques and comprises the following operably linked elements:
- a bacterial replication of origin (ColE1) and antibiotic resistance gene (ampR) allowing efficient propagation and manipulation of the plasmid in bacteria;
- a recombinant retroviral genome comprising, flanked by two LTR sequences,
   - a packaging sequence and,
   - a bicistronic unit comprising the HSV1-TK-encoding nucleic acid and a Thy-1 DNA, separated by an IRES sequence. More particularly, the Thy-1 DNA has been cloned as a XhoI-EcoRI fragment by PCR from a Thy-1 positive cell, and comprises the nucleotide sequence encoding the peptide signal (amino acids -19 to -1) and the entire mature polypeptide (amino acids +1 to +142), including the GPI anchoring domain (amino acid residues 123-142).

Starting from plasmid pKM4, derivatives thereof are being constructed in which the bicistronic unit comprises a chimeric marker DNA comprising a tag inserted linked to Thy-1 or a variant thereof.

More particularly, a first chimeric marker DNA comprises a nucleotide sequence encoding the following domains: (i) the Thy-1 signal peptide (residues - 19 to -1), (ii) the c-myc tag and (iii) the Thy-1 protein (residues +1 to +142) (see Figure 6).

A second chimeric marker DNA comprises a nucleotide sequence encoding the following domains: (i) the Thy-1 signal peptide (residues -19 to -1), (ii) the c-myc tag and (iii) the Thy-1 GPI anchoring domain (residues +123 to +142) (see Figure 6).

### D2: Construction of the replication-defective recombinant retroviruses (Figure 6)

In order to produce the recombinant retroviral vectors, Plasmid pKM4 (see Example D1 above) and the derivatives thereof comprising chimeric marker DNAs are transfected in retroviral packaging cells (see Example A1), in particular in the TE-FLY cells, and the retroviruses produced or the supernatants are recovered. The structure of the genome of the recombinant retroviruses is represented on Figure 6.

### D3: Infection of mature T-lymphocytes

Purified T lymphocytes were prestimulated during 3 days in the presence of OKT3 coated monoclonal antibodies (Orthoclone, Jansen-Cilag) and recombinant human IL-2 (Chiron). Then, the cells were infected with supernatant harvested from TE-FLY-Thy1/1RES/TK packaging cells that produce retroviral particles containing the Thy-1 and TK genes. Infection was performed by centrifugation of 2 x 10⁵ cells/ml of retroviral supernatant at 2400 g during 2 hours, this procedure being repeated three times the same day. Control (mock infection) was performed by manipulating the cells under the same conditions as above using supernatant from TE-FLY parental cells.

The results obtained show efficient delivery of the bicistronic unit to the T lymphocytes, which express the Thy-1 molecule at their surface. This confirms the biological activity of the bicistronic unit and the efficacy of the present method. Furthermore, in the mock infected cells, expression of Thy-1 was very low (i.e., background signal of 1.2%) which further confirm the operability of thy-1 as a marker gene. As indicated above, the Thy-1 molecule can be used to select the cell population that indeed contain and express the transgene, as well as to detect the cells upon in vivo administration.

### E. Example 5 : Construction of a truncated POL construct

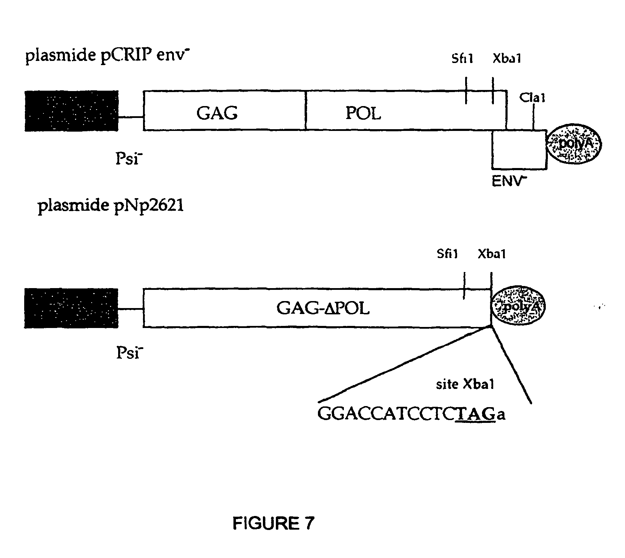

In order to prepare a nucleic acid construct encoding a functional truncated POL protein, plasmid pCRIPenv- was digested with Xbal to generate plasmid pNp2621 (see Figure 7). The sequence of the entire GAG-ΔPOL encoding nucleic acid molecule is represented on Figure 8. The capacity of the resulting constructs to produce retroviral particles was determined in a cell line expressing the env gene and transfected with a retroviral vector containing the LacZ gene (cell line TeIFBasalf). The retrovirus titer was determined, in duplicate, 72h post infection of NIH 3T3 cells. The results obtained are presented in the following Table:

| Plasmid | Titer (p/ml) | |
|---|---|---|
| | Experiment #1 | Experiment #1 |
| pCRIPenv- | 10³ | 6.1X10³ |
| pNp2621 | 2X10³ | 5.6X10³ |

These results demonstrate that the truncated POL protein is biologically active, i.e., retains the capacity to transcomplement defective retroviral genomes.

### F. Example 6 : Retroviral-mediated gene transfer into T-cells

### F1. Materials and methods

Isolation, Culture, and Retroviral Infection of T-cells.

Freshly harvested blood cells were obtained from normal blood donors according to institutional guidelines. CD4⁺, CD8⁺ and total T-cells were isolated from mononuclear cells by negative selection affinity columns (R&D systems, Minneapolis, MN). Cultures of T-cells were performed using clinical grade reagents. Briefly, cells were plated onto 6-well tissue culture plates (Costar, Cambridge, MA) previously coated with anti-CD3 mAb (OKT3 clone, Orthoclone, Jansen-Cilag, Paris, France) at a final concentration of 5 µg/ml. Therefore, cells were grown at 10⁶ cells/ml in RPMI 1640 medium (GIBCO BRL), Grand Island, NY) supplemented with 2 mM L-Glutamine, 100 U/ml penicillin, 100 µg/ml streptomycin and 10% heat-inactivated pooled human AB serum (hABS), supplemented with 600 U/ml IL-2 (Chiron Corporation, Emeryville, CA) for 3 or 5 days until retroviral infection. The day prior the transduction protocol, medium change was performed and activated T-cells were split. For retroviral transduction, TE-FLY GA18 and TE-FLY MOSALF packaging cells containing the Escherichia Coli β-galactosidase (β-gal) gene, described elsewhere (Movassagh M. et al, (1998); (Movassagh, M. et al (1999), were used. Cells were transduced by centrifugation with fresh retroviral supernatant containing 600 U/ml IL-2 and 4 µg/ml protamine sulfate at 2400 x g for 2 h at 30°C. The transduction process was repeated 3 times over a 24 hour-period. Retroviral transduction efficiency was assessed by FDG and X-gal staining (Movassagh M. et al, (1998); (Movassagh, M. et al (1999) on T-cells harvested from suspension cultures 48-hours after the transduction protocol and at different periods of time until 8 week.

### Cell cycle analysis.

Cell cycle analysis of T lymphocyte subsets was performed by DNA staining with propidium iodide using Coulter Reagents Kit (Coultronics, Margency, France). The percentage of T-cells in the different phases of the cell cycle was analyzed using a Multicycle AV software (Phoenix Flow Systems Inc, Seattle, WA).

More specifically, cell cycle was studied by flow cytometry after propidium iodide (PI) staining using Coulter Reagents Kit (Coultronics) that contains reagents for cell lysis and permeabilization (DNA-Prep LPR) and reagents for DNA staining with propidium iodide (DNA-Prep Stain). Briefly, 5 x 10⁵ T-cells were washed after activation in 2 ml PBS, resuspended in DNA-Prep LPR reagent. Then, DNA-Prep stain reagent was added and the cells were incubated in the dark at room temperature for 1 hour. Cell cycle analysis was performed on an EPICS Elite cytometer (Coultronics) on the basis of light scatter properties and fluorescence intensity. Doublets were excluded by plotting peak fluorescence versus integral fluorescence of PI. At least 30,000 events were analyzed at low speed (100 events/second) and collected on listmode files.

### Cell proliferation.

T-cell proliferation was assessed every 24 hours from day 1 to day 8 by tritiated thymidine (³H-Tdr) incorporation. T-cells were pulsed with ³H-Tdr (Amersham, Buckinghamshire, UK; specific activity; 25 Ci/mmol, 1 Mci/ml) for 16 hours and ³H-Tdr incorporation was measured using a β-counter.

### T cell receptor CDR3 size analysis.

Experiments were performed as described (Pannetier, C. et al. (1993). Briefly, RNA was reverse transcribed and a quantity of cDNA corresponding to 300 ng of total RNA was amplified by the polymerase chain reaction (PCR) using a Vβ family-specific primer and a common Cβ primer (Genset, Paris, France) as described (Puisieux, I. et al. (1994). Each Vβ-Cβ PCR product was then subjected to 10 run-off cycles primed with a ROX-labeled internal Cβ primer (Genset) as described(Puisieux, I. et al. (1994). Each run-off product was denatured and loaded on a sequencing gel for fluorescence analysis on a ABI377 DNA sequencer (Perkin Elmer, Norwalk, CT). Data were analyzed with the Immunoscope 3.01 b software (Loginserm, Paris, France) (Pannetier, C. et al. (1993).

### FDG and X-gal staining

For FDG staining, cells after staining either with anti-CD4-PE, CD8-PE or CD3-PE mAbs were counted and brought to 10⁷ per ml in RPMI containing 2% (vol/vol) FCS, 10 mM Hepes (pH 7.3) and 100 µl of 2 mM FDG in H20, prewarmed to 37°C and were added to a 100 µl solution of cells. The cells were mixed gently but thoroughly and rapidly placed back into a 37°C water bath for 10 minutes. Then the cells were placed on ice and 1,800 µl of ice-chilled isotonic incubation medium were added. After 25 minutes, the reaction was stopped by the addition of 1 mM phenylethyl β thiogalactoside (PETG, Sigma). The cells were analyzed by flow-cytometry, 30,000 events being collected in listmode files.

For X-gal staining, cells were fixed in 1 ml PBS containing 1 % glutaraldehyde, 02% formaldehyde for 5 minutes, washed twice in phosphate buffer and then stained for 12 hours at 37°C with 1 ml of X-gal staining solution containing 5 mM ferricyanin, 5 mM ferrocyanine, 2 mM MgCl₂ and 1 mg/ml 5-bromo-4-chloro-3-indoyl-β-D- galactopyranoside as described by Movassagh et al (1998). Only nuclear staining was scored positive.

### F2: RESULTS

### T-cell activation: kinetics of cycling and proliferation

Efficiency of retroviral-mediated gene transfer into T-cells mainly depends on their cycling status (Roe et al., (1993). The induction of cycling for total T-cells ( • ) and for purified CD4^{+ (◊)} or CD8^{+ (□)} - cell subsets was compared. T-cell activation was obtained with clinical grade CD3 mAb and high rhIl2 concentrations. Cell cycle analyses revealed different kinetics of cycling for each T-cell subset (Fig. 9). Indeed, total T-cells and CD8⁺ T-cells enter into S-phase after 48 hours, the maximum percentage of cells in S-phase being reached at day-4 and remaining stable until day 7. Cycling activation of CD4⁺ T-cells appeared slower, the maximum percentage of CD4⁺ cells in S-phase being only reached after 6 days of stimulation. Cell proliferation kinetics were consistent with cell cycle data, although the maximum proliferation assessed by this method was delayed by 24-hours (Figure 16).

Daily monitoring of culture of unfractionated T-cells showed that the CD4/CD8 ratio was reversed after 4 days (Fig. 11). Thus, CD8⁺ cells are triggered into S-phase earlier than CD4⁺ cells leading to an inversion of the CD4/CD8 ratio in the total T-cell population.

### Retroviral-mediated gene transfer into CD4⁺ T-cells.

Based on these results, purified CD4⁺ T-cells were activated by OKT3 mAb and IL-2 for 5 days and subsequently infected on day 6 with GALV β-gal retroviral supernatant using 3 cycles of infection/centrifugation over a 24-hour period. This procedure led to a transduction efficiency of 94.5± 1.5% (Table 10). Quantative evaluation of the frequency of transgene-expressing cells yielded similar results when assayed using X-gal staining (Fig. 13A-B) or FDG staining (Fig. 13C-D). A 24-hour prolongation of the transduction protocol did not result in an improvement of the CD4⁺ T-cells transduction (Table 10).

### Transduction and in vitro expansion of total T-cells, and CD4⁺ and CD8⁺ T-cell subsets.

The transduction protocol optimized for CD4⁺ T-cells was then evaluated to determine whether it could also be efficient for CD8⁺ and total T-cells. For this, total T-cells were activated during either 3 (total T_{d3}) or 5 (total T_{d5}) days - in order to evaluate to what extent the timing of activation might bias the transduction of one or the other T-cells subsets -while CD8⁺ T-cells and CD4⁺ T-cells were activated during 3 or 5 days, respectively.

After retroviral infection, T-cells were expanded in liquid cultures and the percentage of transduced cells was monitored up to 8 weeks. More than 94% of CD4⁺, CD8⁺, total T_{d3} and total T_{d5} cells were transduced (Fig. 14). In most cases, β-gal transgene expression was maintained over 90% during the first 4 weeks. Thereafter, transgene expression progressively decreased in some T-cell cultures. Immunophenotyping of total T-cell cultures indicated that the CD4/CD8 ratio was reversed since day 5 and then remained stable at 0.35-.0.65 (Fig.12). Furthermore, double staining of CD4⁺ or CD8⁺ T-cells with FDG indicated that both subsets equally expressed β-gal activity within the total T-cell cultures, whatever the timing of activation of the total T-cells (data not shown). Thus, transduction of total T-cells after 3 or 5 days of activation leads to similar transduction levels.

Propagation of transduced T-cells in liquid cultures shows that each T-cell subset could be expanded up to 600-1,200-fold after 4 weeks of culture (Table 11). Of note, no toxic effect of β-gal was observed on transduced T-cells.

### T-cell repertoire diversity

Immunoscope analysis was performed on transduced and mock-infected T-cells at different time points up to week 8. The T-cell repertoire was progressively altered, beginning after 2-4 weeks of in vitro culture (Fig. 15). Indeed, the profiles showed restriction of the initial diversity, as observed by loss of the Gaussian distribution of CDR3 lengths to an aspect of oligoclonal T-cell expansions in the majority of the 13 Vβ families studied. These alterations affected both CD4⁺ and CD8⁺ T-cell subsets. Since mock-infected T-cells exhibited similar time-dependent changes, these findings are likely to be secondary to the culture process but not the retroviral transduction perse.

### F3. Discussion

Gene transfer into total T-cells and purified CD4⁺ and CD8⁺ subsets to unprecedented levels by using pseudotyped retroviral vectors and a simple 24-hour transduction protocol optimized following cell cycling analysis of T-cell activation has been achieved. Because proviral integration of MLV-derived vectors depends on the cycling status of target cells (Roe et al 1993), most of the T-cell infection protocol developed to date include an activation step of 2 to 3 days (see, for example Bunnell et al., (1995). Cell cycle analyses showed that the majority of CD4⁺ cells can be triggered into S-phase following activation by anti-CD3 in the presence of IL-2 for 5 days whereas CD8⁺ T-cells reach maximum proliferation earlier. Consequently, a relatively short timing of activation (i.e., ≤ 3 days), albeit sufficient to transduce CD8⁺ T-cells is obviously not optimal for transducing CD4⁺ T-cells.

The different cycling kinetics of CD4⁺ and CD8⁺ T-cell subsets presumably participates to the imbalance of the CD4/CD8 ratio after T-cell stimulation was observed. Thus, cultures of total T-cells infected after 3 or 5 days of activation yields to a clear predominance of transduced CD8⁺ cells. This result, which is in accordance with another published report (Maviolio et al (1994)), may have significant clinical consequences. Indeed, after T-cell depleted bone marrow transplantation, CD4 T-cells recovery is significantly longer to achieve than CD8 T-cell recovery (Small et al (1999)). Thus, the clinical use of CD8-biased T-cell populations together with hematopoietic stem cells may alter the T-cell reconstitution.

Until recently, retroviral transduction of hematopoietic progenitors and T-cells has remained relatively low. The development of packaging cell lines producing pseudotyped virions and the use of artifices such as the centrifugation of the target cell with a viral supernatant (Bunnell et al (1995)) and/or the colocalization of virions and target cells on fibronection (FN) fragments (Hanenberg et al (1996)) have improved the efficiency of the gene transfer into hematopoietic cells. Using these techniques it has been shown that hematopoietic progenitors and dendritic cells can be transduced at very high levels by using GALV-pseudotyped vectors and a transduction protocol combining cell centrifugation and FN, as demonstrated above.

Recently, high transduction efficiency of T-cells using amphotropic vectors and FN fragments have also been reported by repeatedly infecting T-cells with viral supernatant collected at 32°C for 2-3 days (Pollock et al (1998)). Under these conditions, 80% to 87% of T cells could be transduced. However, this method remains tine-consuming because multiple days of infection are required. Surprisingly, FN alone or combined with cell centrifugation did not enhance the results.

Thus, the infection protocol was shortened to 24 hours by using GALV-pseudotyped vectors and cell centrifugation only, as demonstrated above. It is known that human T-cells express higher levels of GLVR-1 mRNA than GLVR-2 mRNA, coding for the GALV and amphotropic envelope receptors, respectively. Furthermore, both mRNA are upregulated following growth in IL-2 and anti-CD3 stimulation (Lame et al. (1996)). Although these findings cannot be directly correlated with surface expression of GLVR-1, it turns out that the results indicate that infecting T-cells with GLAV-pseudotyped vectors after activation by high concentrations of IL-2 contributes to higher gene transfer efficiency.

Although a relatively high multiplicity of infection (25 to 50 particles/cell) has been used, 3 consecutive rounds of infection were necessary to achieve high transduction levels. This might be due to the asynchronous cell cycle entry of target T-cells and/or because T-cells may have a relatively low permissivity to retroviral particles.

Since the present methods used clinical grade chemicals, these conditions are directly applicable to the clinic. This represents a major advance in view of developing clinical trials based on the use of genetically engineered T-cells. However, depending on the clinical application it might be important to transduce either a T-cell subset like CD4⁺ T-cell subsets or to select transduced T-cells prior to reinfusion. These results show that it is possible to transduce at the same level either CD4⁺ or CD8⁺ T-cell subsets and to allow their expansion within 4 weeks while maintaining transgene expression. Since most clinical applications require the infusion of 10⁵ to 10⁷ cells/kilogram, the present method should allow the obtention of sufficient numbers of transduced T-cells.

In some later T-cell cultures (> 4 weeks), transgene expression diminished contemporaneously to a decline in T-cell proliferation. This finding is in agreement with recent data showing that T-cell activation modulates retrovirus-mediated gene expression (Quinn et al 1998).

Finally immunoscope analysis of the T-cell repertoire diversity showed that cultures of transduced and non-transduced T-cells severely altered the Gaussian distribution of TCRβ CDR3 lengths after 2 to 3 weeks of expansion. Thus, this phenomenon was not related to the transduction protocol itself, but rather to the duration of culture. This is in accordance with the previous observations that *in vitro* culture promotes oligoclonal T-cell expansions and alters the T-cell repertoire (Schirmbeck et al (1993); Dietrich et al (1997). In these experiments both CD4⁺ and CD8⁺ subsets were effected by these changes. Since CD8⁺ T-cells are more susceptible to clonal expressions *in vivo* than CD4⁺ T-cells, *in vitro* inversion of the CD4/CD8 ratio may represent an additional factor contributing to T-cell repertoire alterations (Wack et al (1998)). The functional consequences of CD4/CD8 imbalance and repertoire restriction on *in vivo* immune responses are unknown at present. Nevertheless, such findings strongly support the need for shortening the duration of *in vitro* T-cell expansion prior to reinfusion in order to preserve the overall quality of the T-cell repertoire to be reinfused.

### G. Example 7 : Retroviral-mediated gene transfer into T-cells

Freshly harvested blood cells were obtained from normal blood donors. Total T-cells were isolated from mononuclear cells by negative selection affinity columns (R&D Systems). T-cells plated onto tissue culture plates previously coated with anti-CD3 monoclonal antibody at 5 µg/ml were grown in RPMI 1640 medium supplemented with 10% heat inactivated pooled human AB serum and 600 units/ml interleukin2 for 5 days. Thereafter, T-cells were retrovirally transduced using supernatant for TE-FLYGA16 packaging cells. These cells produce 10⁶ virions/ml containing Thy-1 (CD90) gene. Cells were transduced by centrifugation in the presence of interleukin2 and protamine sulfate as described above.

48 hours after retroviral infection, the percentage of CD3⁺ transduced T-cells was analyzed by flow-cytometry after staining of the cells with anti-CD3 and anti-Thy-1 (CD90) monoclonal by antibodies labeled to FITC and phycoerytrin, respectively.

The results of three separate experiments are shown on Table 12.

### REFERENCES

Aicher A, et al., Exp Hematol 25:39-44, 1997
Bello-Fernandez C, et al., Gene Therapy 8:1651-1658, 1997
Bunnell, et al., Proc Natl Acad Sci USA 92, 7739-7743 (1995)
Chuck, A.S et al., Hum. Gen. Ther. 7, 743-756
Caux C, et al., Nature 360:258-261, 1992
Chapuis F, et al., Eur. J. Immunol. 27:431-437, 1997
Champseix C, et al., Blood 88:107-113, 1996
Dietrich, P.Y. et al., Int Immunol 9, 1073-1083 (1997)
Germeraad, W.T.V., et al., Blood 84, 780-788
Gluckman, E. et al., J. Med. 321, 1174-1178
Guigon M, et al., Blood 90:3364a, 1997 (abstr)
Guery JC, et al., T cells. J Immunol 154:536-44, 1995
Hanenberg, H. et al, Nature Med. 2, 876-882 (1996)
Hatzfeld, A., Hum. Gene Ther. 7, 207-213
Hugues, P.F.D. et al., Blood 74, 1915-1922
Issaad, C., et al., Blood 81, 2916-2924
Lame et al., Hum Gene Ther 17 (1415-1422 (1996)
Lu., L., et al., J. Exp. Med. 178, 2089-2096
Mayordomo Jl, Nature Med 1:1297-1302, 1995
Mavilio, F. et al., Blood 83, 1988-1997 (1994)
Mayani, H., et al., Exp. Hematol. 23, 1453-1462
Moore, K.A., et al., Blood 79, 1393-1399
Moritz, T., et al., J. Exp. Med. 178, 529-536
Moritz, T., et al., J. Clin. Invest. 93, 1451-1457
Moritz, T., et al., Blood 88, 855-862
Movassagh, M., et al., Blood 88, 1277-1283
Movassagh, M., et al., 1997, Stem Cells 15, 214-222
Movassagh, M. et al., Hum Gene Ther 9, 225-234 (1998)
Movassagh, M. et al., Hum Gene Ther 10, 175-187 (1999)
Nolta, J.A., et al., Exp. Hematol. 20, 1065-1071
Pannetier, C. et al., Proc Natl Acad Sci USA 90, 4319-4323 (1993)
Planelles et al., Gene Ther. 2, 369-376, 1995
Pollok, K et al., J Virol 72, 4882-4892 (1998)
Porter, C.D., Hum. Gene Ther., 7 913-919.
Puisieux, I. et al., J Immunol 153, 2807-2818 (1994)
Quinn, E.R., et al., Hum Gene Ther 9, 1457-1467 (1998)
Roe, T. et al., EMBO J 12, 2099-2108 (1993)
Romani N, et al., J. Exp. Med. 180:83-93, 1994
Rusten LS, et al., Blood 87:1317-1325, 1996
Seki et al., PNAS 82:6657-6661, 1985
Schirmbeck, R. et al., Cell Immunol 149, 444-449 (1993)
Small, T.N. et al., Blood 93, 467-480 (1999)
Szabolcs P, et al., Blood 90:2160-2167, 1997
Wack, A. et al., Int Immunol 10, 1281-1288 (1998)
Young JW, et al.; J Exp Med 183:7-11, 1996
Zitvogel L, et al., J. Exp. Med 183:87-97, 1996

**TABLE 1**

| Effect of time and number of cycles on the percentage of transduction of CFC and LTC-IC-derived colonies using centrifugation and adhesion to fibronectin | | | | | | |
|---|---|---|---|---|---|---|
| | Centrifugation + fibronectin | | | | | |
| | **WITHOUT POLYBRENE** | | | **WITH POLYBRENE** | | |
| | 24 hr | 48 hr | 72 hr | 24 hr | 48 hr | 72 hr |
| BFU-E | 30 ± 5 | 41 ± 5^{a} | 40 ± 6ₐ | 38 ± 3 | 29 ± 1 | 31 ± 5 |
| CFU-GM | 17 ± 3 | 29 ± 4^{a} | 30 ± 3^{a} | 21 ± 0.6 | 23 ± 2 | 21 ± 1 |
| Total progenitors | 24 ± 4 | 34 ± 4^{a} | 33 ± 2^{a} | 28 ± 1 | 25 ± 2 | 23 ± 1 |
| LTC-IC-derived colonies | 21 ± 2 | 22 ± 2 | 23 ± 1 | 24.5 ± 1 | 10 ± 2 | 11 + 5 |

| | | | | | | |
|---|---|---|---|---|---|---|
| Results are the mean ± SEM of at least three separate experiments ^{a}p < 0.05 (paired t-test) | | | | | | |

**TABLE 2**

| Gene Transfer Efficiency on the transduction of CD34+ cells, CFC and LTC-IC-derived colonies using GALV-Pseudotyped Retroviral Vectors | | | |
|---|---|---|---|
| Percentage of transduction | | | |
| Experiments | CD34⁺ cells | CFC | LTC-IC-derived colonies |
| 1 | ND | 82 | 73 |
| 2 | 69 | 87 | 55 |
| 3 | 47 | 65 | 60 |
| 4 | 42 | 97 | 66 |

| | | | |
|---|---|---|---|
| ND, Not done | | | |

**TABLE 3**

| DETECTION OF NLS-LACZ GENE BY PCR ANALYSIS OF INDIVIDUAL CFC AND LTC-IC-DERIVED COLONIES | | | | | |
|---|---|---|---|---|---|
| **EXPERIMENTS** | **CFU-GM** | **CFU-M** | **BFU-E** | **TOTAL CFC (%)** | **LT-IC-DERIVED COLONIES (%)** |
| 1 | 5/5 | 5/5 | 5/5 | 15/15 (100) | 10/100 (100) |
| 2 | 10/10 | 10/10 | 10/10 | 30/30 (100) | 10/10 (100) |
| 3 | 9/10 | 9/10 | 8/9 | 26/29(90) | 9/10 (90) |
| 4 | 9/9 | 8/9 | 10/10 | 27/28 (96) | 10/10 (100) |

| | | | | | |
|---|---|---|---|---|---|
| Numbers represent the number of PCR-positive colonies/total number of colonies evaluated. 0, percent of positive colonies Detection of nls-LacZ gene integration was studied on DNA extracted from individual CFC and from individual LTC-IC-derived colonies. Presence of DNA has been confirmed using PCR with β-globin primers. | | | | | |

**TABLE 4**

| **mCD2 (A) and nls-LacZ expression (B) by cord blood cells after infection with MFG-mCD2 amphotropic retrovirus or nls-LacZ GALV-pseudotyped retrovirus** | | | | | |
|---|---|---|---|---|---|
| A | Percentage of mCD2 tranduced cells | | | | |
| | exp.1 | exp.2 | exp.3 | exp.4 | Mean ± sem |
| Total mCD2⁺ cells | 28 | 37 | 53 | 38 | 39 ± 5 |
| CD34⁺ mCD2⁺ cells | 33 | 37 | 52 | 37 | 40 ± 4 |

| B | Percentage of b-gal tranduced cells | | | | |
|---|---|---|---|---|---|
| | exp.1 | exp.2 | exp.3 | exp.4 | Mean ± sem |
| Total b-gal⁺ cells | 61 | 58 | 67 | 64 | 62.5 ± 2 |
| CD34⁺ b-gal⁺ cells | 71 | 75 | 77 | 66 | 72 ± 2 |

| | | | | | |
|---|---|---|---|---|---|
| Transgene expression was studied by FACS analysis on at least 2 x 10⁴ cells 18-24 hours after infection. For mCD2 gene expression, CD34⁺ cells infected by MFG mCD2 supernatants or mock infected by NIH-3T3 supernatants were double stained with mCD2-FITC and CD34-PE-Cy5 mAbs. For nls-LacZ gene expression, CD34⁺ cells infected by G18 GALV pseudotyped viral supernatants or mock infected by T-MOSALF supernatants were stained with CD34-PE-Cy5 mAbs and then with FDG used as substrate for the detection of b-gal activity. | | | | | |

**TABLE 5**

| **Effects of various growth factors combinations on the generation in liquid culture of CD1a⁺ DC derived from mCD2⁺ transduced CD34⁺ cells** | | | |
|---|---|---|---|
| | | Percentage of CD1a⁺ cells | Total number of cells (x10³) |
| n= 3 | GM-CSF + IL-4 + SCF + TNFa | 69 ± 4 | 240 ± 120 |
| | GM-CSF + IL-4 + SCF + TNFa + FL (50) | 70 ± 3 | 610 ± 180^{‡} |
| | GM-CSF + IL-4 + SCF + TNFa + FL (300) | 68 ± 0.3 | 410 ± 190^{‡} |
| | | | |
| *n*= 5 | GM-CSF + IL-4 + SCF + FL (50) | 49 ± 8 | 1800 ± 600 |
| | GM-CSF + IL-4 + SCF +FL (50) + TNFa | 73 ± 5^{‡} | 1000 ± 400^{‡} |

| | | | |
|---|---|---|---|
| CD34⁺ cells were infected by MFG-mCD2 supernatants and then sorted into transduced mCD2-positive fraction. Sorted fractions, including CD34⁺ cells mock-infected by NIH-3T3 supernatants, were cultured in the presence of various growth factors combinations. After 12 days of culture, cells were counted and the percentage of CD1a⁺DC was evaluated by FACS analysis on at least 2 x 10⁴ cells. Results represent the mean ± sem of (n) experiments for 2 x 10⁴ CD34⁺ cells. ‡ statistically significant by paired t-test : p<0.05 | | | |

**TABLE 6**

| **Gene transfer efficiency on dendritic cells derived from liquid culture of CD34⁺ cells transduced with MFG-mCD2 amphotropic retrovirus (A) or nls-LacZ GALV-pseudotyped retrovirus (B)** | | | | | | |
|---|---|---|---|---|---|---|
| **A** | **Percentage of mCD2⁺ cells derived from sorted CD34⁺ / mCD2⁺ cells** | | | | | |
| | exp.1 | exp.2 | exp.3 | exp.4 | exp.5 | Mean ± sem |
| Total mCD2⁺ cells | 36.5 | 39 | 62 | 37 | 73 | 49 ± 7 |
| CD1a⁺ mCD2⁺ cells | 58 | 51 | 64 | 47 | 77 | 59 ± 5^{‡} |
| | | | | | | |

| **B** | **Percentage of b-gal⁺ cells derived from sorted CD34⁺ / b-gal⁺ cells** | | | | | |
|---|---|---|---|---|---|---|
| | exp.1 | exp.2 | exp.3 | exp.4 | exp.5 | Mean ± sem |
| Total b-gal⁺ cells | 88 | 64 | 64.5 | 64 | 69 | 70 ± 5 |
| CD1a⁺ b-gal⁺ cells | 89 | 73 | 66 | 71.5 | 72 | 74.5 ± 4^{‡} |

| | **Percentage of b-gal⁺ cells derived from total infected CD34⁺ cells** | | | | | |
|---|---|---|---|---|---|---|
| | exp.1 | exp.2 | exp.3 | exp.4 | Means ± sem | |
| Total b-gal⁺ cells | 53 | 46 | 49 | 62 | 52.5 ± 3 | |
| CD1a⁺ b-gal⁺ cells | 65.5 | 83 | 62.5 | 83 | 73.5 ± 5^{‡} | |

| | | | | | | |
|---|---|---|---|---|---|---|
| CD34⁺ cells after infection by MFG-mCD2 or by G18 supernatants were sorted into transduced mCD2 or β-gal positive fractions. Sorted fractions including mock-infected (NIH-3T3 supernatants or T-MOSALF supernatants) CD34⁺ cells were cultured in the presence of GM-CSF + IL-4 + SCF + TNFa + FL (50) during 12 days. Then, cells were counted and the percentage of transduced and non-transduced CD1a⁺DC was evaluated by FACS analysis on at least 2 x 10⁴ cells. For mCD2 gene expression, cells were double stained with mCD2-FITC and CD1a-PE mAbs. For nls-LacZ gene expression, cells were stained with CD1a-PE mAbs and then with FDG used as substrate for the detection of b-gal activity. ^{‡} Differences between the percentage of CD1a⁺ transduced cells and the percentage of total transduced cells were statistically significant by paired *t*-test, p<0.05. | | | | | | |

**TABLE 7**

| **Immunophenotypic characteristics of CD1a⁺ dendritic cells derived from liquid culture of CD34⁺ cells transduced with MFG-mCD2 amphotropic retrovirus or with nls-LacZ GALV pseudotyped retrovirus** | | | |
|---|---|---|---|
| | Transduced CD1a⁺ cells^{†} | | Non-transduced CD1a⁺ cells |
| | MFG-mCD2 | Nls-LacZ GALV | |
| | (n = 7) | (n = 4) | (n = 7) |
| CD1a / HLA-DR | 86 ± 6 | 94 ± 2 | 90 ± 3 |
| CD1a / CD40 | 94 ± 2 | 83 ± 9 | 87 ±3 |
| CD1a / CD54 | 79 ± 5 | 78 ± 8 | 70 ± 8 |
| CD1a / CD80 | 70 ± 5 | 65 ± 12 | 69 ± 6 |
| CD1a / CD86 | 33 ± 7 | 32 ± 9 | 38 ± 5 |

| | | | |
|---|---|---|---|
| CD34⁺ cells after infection by MFG-mCD2 supernatants or by G18 supernatants were sorted into transduced mCD2 or β-gal positive fraction. Sorted fractions including mock-infected (NIH-3T3 supernatants or T-MOSALF supernatants) CD34⁺ cells were cultured in the presence of GM-CSF + IL-4 + SCF + TNFa + FL (50) during 12 days. Then, cells were counted and the immunophenotypic characteristics of transduced and non-transduced CD1a⁺DC were evaluated by FACS analysis on at least 2 x 10⁴ gated CD1a⁺ cells. Results are expressed as percentage of double positive cells and represent the mean sem of (n) experiments. ^{†}: Comparisons of percentage of transduced CD1a⁺ cells to non-transduced CD1a⁺ cells were not statistically significant by non-paired t-test, p>0.1. | | | |

**TABLE 8**

| **Transduction of clonogenic dendritic progenitors using nls-LacZ GALV-pseudotyped retrovirus** | | | | |
|---|---|---|---|---|
| | T-MOSALF ecotropic retroviral infections (mock) | G18 GALV-pseudotyped retroviral infections | | |
| | Number of colonies* | Number of colonies^{‡} | % of β-gal- positive colonies | % of PCR-positive colonies |
| CFU-DC | 13 ± 5 | 18 ± 5 | 96 ± 1 | 100 (40/40) |
| CFU-GM/DC | 41 ± 8 | 37 ± 7 | 94 ± 1 | 95 (37/39) |
| CFU-GM | 27 ± 5 | 20 ± 4 | 89 ± 3 | 92 (35/38) |

| | | | | |
|---|---|---|---|---|
| CD34⁺ cells were mock-infected with T-MOSALF supernatants or infected with G18 supernatants and then plated in methylcellulose under conditions allowing the development of clonogenic dendritic progenitors within 12 days of culture (see Materiel and methods). β-gal activity and provirus integration were studied on individual colonies using X-gal staining and PCR analysis, respectively. Results are the mean ± sem of five separate experiments. ( ) : Number of individual PCR-positive colonies / total of colonies evaluated. * : Number of colonies for 4 x 10³ plated CD34⁺ cells. ^{‡} : Comparison of number of transduced colonies to mock-infected colonies was not statistically significant by paired t-test, p>0.1. | | | | |

**TABLE 9**

| **Transfer of genes and CD4+ lymphocytes** | | | | | | |
|---|---|---|---|---|---|---|
| | | 0 | 2 | 4 | 6 | 8 |
| Exp. 1 | % of transduction | 98 | 94,5 | 90,3 | 94,3 | |
| | Number of cells | 10⁶ | 1,1-10⁸ | 2,2-10⁸ | 3,1-10⁸ | |
| | | | | | | |
| Exp. 2 | % of transduction | 97,7 | 98,5 | 98 | 77 | 99 |
| | Number of cells | 10⁶ | 4,5.10⁷ | 7,8.10⁸ | 6,5.10⁹ | 6,7.10¹¹ |
| | | | | | | |
| Exp. 3 | % of transduction | 99,3 | 97,5 | 93 | 100 | 96,5 |
| | Number of cells | 106 | 4.108 | 8.109 | 1,8.1010 | 1,4.1011 |

**TABLE 10**

| **Table 11: Gene transfer efficiency into CD4 T-cells after 24 and 48 hours of infection** | | |
|---|---|---|
| | Percent of CD4⁺ T-cells expressing β-galactosidase activity | |
| Experiment | 24 hours | 48 hours |
| 1 | 96 % | 97 % |
| 2 | 92 % | 98 % |
| 3 | 93 % | 96 % |
| 4 | 98 % | 91 % |
| Mean ± sem | 94 % ± 1.5 | 95 % ± 1.5 |

**TABLE 11**

| Total cell number after in vitro expansion of different transduced T-cell populations | | | | |
|---|---|---|---|---|
| | WEEKS | | | |
| | 0 | 2 | 3 | 4 |
| CD4⁺ T-cells | 1.10⁶ | 9.8 ± 1.1 x 10⁷ | 4.6 ± 1.5 x 10⁸ | 1 ± 0.4 x 10⁹ |
| (n = 4) | | | | |
| CD8⁺ T-cells | 1.10⁶ | 10.7 ± 6.1 x 10⁷ | 4.3 ± 1.7 x 10⁸ | 1.2 ± 0.7 x 10⁹ |
| (n = 3) | | | | |
| Total T_{d3} cells | 1.10⁶ | 13.2 ± 0.8 x 10⁷ | 3.8 ± 2.7 x 10⁸ | 0.9 ± 0.5 x 10⁹ |
| (n=4) | | | | |
| Total T_{d5} cells | 1.10⁶ | 2.5 ± 0.7 x 10⁷ | 0.9 ± 0.3 x 10⁸ | 0.6 ± 0.1 x 10⁹ |
| (n = 4) | | | | |

| | | | | |
|---|---|---|---|---|
| Results are the mean ± sem of (n) experiments. | | | | |

**TABLE 12**

| **Transduction of T lymphocytes with GALV-retrovirus vectors carrying Thy-1 genes** | |
|---|---|
| | % of CD3⁺ CD90⁺ cells |
| Exp 1 | 23,3 |
| Exp 2 | 42,1 |
| Exp 3 | 37,2 |
| Mean ± SEM | 34.2 ± 5.6 |

## Claims

1. A method of delivery of a nucleic acid to hematopoietic cells in vitro or ex vivo, wherein said method comprises:
a) providing a population of hematopoietic cells,
b) contacting said population of hematopoietic cells with a defective recombinant retrovirus, wherein said retrovirus:
- is pseudotyped with a GALV envelope, and
- comprises said nucleic acid,
c) subjecting the cell population to a centrifugation step; and
d) collecting the cell population obtained in step c),
wherein the retrovirus further comprises:
- a marker nucleic acid, coding for a membrane polypeptide comprising an extracellular domain anchored in or at the membrane, but lacking an intracytoplasmic domain;
- a bicistronic unit comprising said nucleic acid and said marker nucleic acid operably linked by an IRES sequence;
and wherein said defective recombinant retrovirus is produced in a packaging cell line comprising a truncated retroviral pol DNA.

2. The method of claim 1, wherein, in c), the cell population is subjected to a centrifugation step and a fibronectin adhesion step.

3. The method of claim 1 or 2, wherein, between (c) and (d), the cell population is contacted again with a defective recombinant retrovirus as defined in step (b).

4. The method of claim 1, wherein said membrane polypeptide is human Thy-1.

5. The method of claim 1 or 4 wherein said membrane polypeptide comprises a tag.

6. The method of claim 1, wherein the centrifugation is carried out in the presence of a polycation, such as polybrene or protamine sulfate.

7. The method of claim 1 or 6, wherein the centrifugation is performed between 100 to 3000 g.

8. The method of claim 2, wherein the fibronectin adhesion step is performed by incubating the cell population on a support coated with human fibronectin or fragments thereof.

9. The method of claim 1, wherein the hematopoietic cells are immature hematopoietic cells.

10. The method of claim 1, wherein said hematopoietic cells are selected from T lymphocytes, B lymphocytes, monocytes and dendritic cells.

11. The method of claim 9, wherein after step (d) said hematopoietic stem cells are further differentiated into dendritic cells.

## Patentansprüche

1. Ein Verfahren zur Zufuhr einer Nukleinsäure in vitro oder ex vivo an hämatopoetische Zellen, wobei das Verfahren die folgenden Schritte umfasst:
a) Bereitstellen einer Population hämatopoetischer Zellen,
b) In Kontakt bringen dieser Population hämatopoetischer Zellen mit einem defekten rekombinanten Retrovirus, wobei der Retrovirus:
- mit einer GALV Hülle pseudotypisiert ist, und
- diese Nukleinsäure umfasst,
c) Unterziehen der Zellpopulation eines Zentrifugationsschritts; und
d) Sammeln der Zellpopulation, die im Schritt c) erhalten wird,
wobei der Retrovirus ferner umfasst:
- eine Marker Nukleinsäure, die für ein Membran-Polypeptid kodiert, umfassend eine extrazelluläre Domäne, die in oder auf der Membran verankert ist, der aber eine intracytoplasmatische Domäne fehlt;
- eine bicistronische Einheit umfassend die Nukleinsäure und die Marker Nukleinsäure, die durch eine IRES Sequenz funktionell verknüpft sind;
und wobei das defekte rekombinante Retrovirus in einer Verpackungszelllinie hergestellt wird, die eine trunkierte retrovirale pol DNA umfasst.

2. Das Verfahren nach 1, wobei die Zellpopulation in c) einem Zentrifugationsschritt und einem Fibronektin Adhäsionsschritt unterzogen wird.

3. Das Verfahren nach 1 oder 2, wobei die Zellpopulation zwischen c) und d) nochmals mit einem defekten rekombinanten Retrovirus wie in Schritt b) definiert, in Kontakt gebracht wird.

4. Das Verfahren nach 1, wobei dieses Membran Polypeptid das humane Thy-1 ist.

5. Das Verfahren nach 1 oder 4, wobei das Membran Polypeptid einen Tag umfasst.

6. Das Verfahren nach 1, wobei die Zentrifugation in Gegenwart eines Polykations, zum Beispiel Polybren oder Protaminsulfat durchgeführt wird.

7. Das Verfahren nach 1 oder 6, wobei die Zentrifugation zwischen 100 bis 3000 g durchgefiihrt wird.

8. Das Verfahren nach 2, wobei der Fibronektin Adhäsionsschritt durch Inkubieren der Zellpopulation auf einem Träger, der mit humanem Fibronektin oder Fragmenten davon beschichtet ist, durchgeführt wird.

9. Das Verfahren nach 1, wobei die hämatopoetischen Zellen unreife hämatopoetische Zellen sind.

10. Das Verfahren nach 1, wobei diese hämatopoetischen Zellen ausgewählt sind aus T-Lymphozyten, B-Lymphozyten, Monozyten und dendritischen Zellen.

11. Das Verfahren nach 9, wobei diese hämatopoetischen Zellen nach Schritt d) weiter zu dendritischen Zellen differenziert werden.

## Revendications

1. Procédé de délivrance d'un acide nucléique à des cellules hématopoïétiques in vitro ou ex vivo, ledit procédé comprenant
a. la fourniture d'une population de cellules hématopoïétiques,
b. la mise en contact de ladite population de cellules hématopoïétiques avec un virus défectif recombinant, où ledit rétrovirus :
- est pseudotypé avec une enveloppe GALV, et
- comprend ledit acide nucléique,
c. la soumission de la population cellulaire à une étape de centrifugation ; et
d. la récupération de la population cellulaire obtenue à l'étape c),
le rétrovirus comprenant en outre :
- un acide nucléique marqueur, codant pour un polypeptide membranaire comprenant un domaine extracellulaire ancré dans ou sur la membrane, mais dépourvu d'un domaine intracytoplasmique ;
- une unité bicistronique comprenant ledit acide nucléique et ledit acide nucléique marqueur liés de manière opérante par une séquence IRES ;
et où ledit rétrovirus défectif recombinant est produit dans une lignée cellulaire d'encapsidation comprenant un ADN pol rétroviral tronqué.

2. Procédé selon la revendication 1, dans lequel, à l'étape c), la population cellulaire est soumise à une étape de centrifugation et à une étape d'adhésion par de la fibronectine.

3. Procédé selon la revendication 1 ou 2, dans lequel, entre (c) et (d), la population cellulaire est mise de nouveau en contact avec un rétrovirus défectif recombinant tel que défini à l'étape (b).

4. Procédé selon la revendication 1, dans lequel ledit polypeptide membranaire est Thy-1 humain.

5. Procédé selon la revendication 1 ou 4, dans lequel ledit polypeptide membranaire comprend une étiquette (tag).

6. Procédé selon la revendication 1, dans lequel la centrifugation est conduite en présence d'un polycation, tel que le polybrene ou le sulfate de protamine.

7. Procédé selon la revendication 1 ou 6, dans lequel la centrifugation est menée entre 100 et 3000g.

8. Procédé selon la revendication 2, dans lequel l'étape d'adhésion par la fibronectine est menée en incubant la population cellulaire sur un support recouvert avec de la fibronectine humaine ou des fragments de celle-ci.

9. Procédé selon la revendication 1, dans lequel les cellules hématopoïétiques sont des cellules hématopoïétiques immatures.

10. Procédé selon la revendication 1, dans lequel lesdites cellules hématopoïétiques sont choisies parmi les lymphocytes T, les lymphocytes B, les monocytes, et les cellules dendritiques.

11. Procédé selon la revendication 9, où, après l'étape (d) lesdites cellules hématopoïétiques souches sont en outre différenciées en cellules dendritiques.
